# EUROPEAN PATENT SPECIFICATION

(11) **EP 1 939 279 B1**
(45) Date of publication and mention of the grant of the patent: **11.07.2012**
(21) Application number: 07122035.4
(22) Date of filing: 30.11.2007
(51) Int. Cl.: G01N 33/50

(54) **Skin model**
Hautmodell
Modèle de peau

(30) Priority: 01.12.2006 EP 06125283
(43) Date of publication of application: 02.07.2008
(73) Proprietor: Symrise AG, 37603 Holzminden (DE); Cutech S.R.L., 35129 Padova (IT)
(72) Inventor: Vielhaber, Gabriele, 37603 Holzminden (DE); Pertile, Paolo, 35020 San Pietro Viminario (IT)
(74) Representative: Fabry, Bernd

(56) References cited:
- WO-A-2004/092726
- JACOBS J J L ET AL: "METHYL GREEN-YPRONINE STAINING OF PORCINE ORGANOTYPIC SKIN EXPLANT CULTURES: AN ALTERNATIVE MODEL FOR SCREENING FOR SKIN IRRITANTS" ATLA. ALTERNATIVES TO LABORATORY ANIMALS, LONDON, GB, vol. 28, 2000, pages 279-292, XP009038188 ISSN: 0261-1929
- GORE ANURADHA V ET AL: "Comparative biomembrane permeation of tacrine using Yucatan minipigs and Domestic pigs as the animal model" JOURNAL OF PHARMACEUTICAL SCIENCES, vol. 87, no. 4, April 1998 (1998-04), pages 441-447, XP002430715 ISSN: 0022-3549
- MOISON RALF M W ET AL: "Topically applied vitamin C and cysteine derivatives protect against UVA-induced photodegradation of suprofen in ex vivo pigskin." PHOTOCHEMISTRY AND PHOTOBIOLOGY, vol. 77, no. 4, April 2003 (2003-04), pages 343-348, XP009082731 ISSN: 0031-8655
- RIJNKELS JOLANDA M ET AL: "TIME AND DOSE-RELATED ULTRAVIOLET B DAMAGE IN VIABLE PIG SKIN EXPLANTS HELD IN A NEWLY DEVELOPED ORGAN CULTURE SYSTEM" PHOTOCHEMISTRY AND PHOTOBIOLOGY, OXFORD, GB, vol. 73, no. 5, 1 May 2001 (2001-05-01), pages 499-504, XP009082730 ISSN: 0031-8655
- MEYER W ET AL: "UNTERSUCHUNGEN ZUR STRUKTUR DER OHRHAUT BEIM HAUSSCHWEIN, UNTER BESONDERER BERUEKSICHTIGUNG EINER VERWENDUNG IN DER HUMANDERMATOLOGISCHEN FORSCHUNG INVESTIGATION IN THE STRUCTURE OF THE EAR SIN IN THE DOMESTICATED PIG, WITH SPECIAL REFERENCE TO THE USE FOR HUMAN DERMATOLOGICAL RESEARCH" BERLINER UND MUENCHENER TIERAERZTLICHE WOCHENSCHRIFT, PAUL PAREY, BERLIN, DE, vol. 114, no. 3/4, 2001, pages 92-111, XP009038241 ISSN: 0005-9366
- MEYER W ET AL: "THE SKIN OF DOMESTIC MAMMALS AS A MODEL FOR THE HUMAN SKIN, WITH SPECIAL REFERENCE TO THE DOMESTIC PIG" CURRENT PROBLEMS IN DERMATOLOGY, KARGER, BASEL, CH, vol. 7, 1978, pages 39-52, XP009033716 ISSN: 1421-5721

## Description

### Field of invention

The present invention relates to the use of a pig skin sample as a skin model, particularly for the assessment of pharmacological and cosmetic effects.

### State of the art

The skin being the largest human organ, all substances intended for topical application thereon must undergo extensive testing to ensure their safety. However, testing substances on the human skin is only possible under a few exceptional circumstances. In the past, testing therefore had to be performed on animals. However, animal testing, i. e. performing tests on the skin of living animals, is undesired or even forbidden by law. There is thus a pressing need to develop skin models which circumvent the need to expose living animals to potentially hazardous substances.

Conventional skin models often rely on reconstituted human or animal skin. Frequently, monolayer cultures of selected skin cell types (e. g. fibroblasts) are grown in a culture medium and are charged with a substance the effects of which are to be assessed. However, such monolayer cultures do not allow assessing effects resulting from interaction of different cell types or resulting from three-dimensional cell interaction. In addition, the time required for establishing such cultures is considerably long. Monolayer skin cell cultures are thus deemed inadequate for faithfully modelling the effects of a substance on human skin.

Other models involve reconstructed skin models, consisting of a maximum of four different cell types, most commonly keratinocytes, melanocytes and fibroblasts (EpiSkin^{™} [L'Oreal, Paris, France], EpiDerm^{™} [MatTek, Ashland, USA], SkinEthic^{™} [SkinEthic, Nice, France]. Although these models are useful to study selected effects in the dermis or epidermis, they are not suitable for studying the whole range of possible interactions of a substance with human skin. Attempts to construct a full skill model in vitro have so far failed.

It has therefore been tried to establish a culturing system for intact skin samples or ex vivo skin samples. As human ex vivo skin samples are only poorly available, either mice or pig ex vivo skin samples are currently used. The use of pig skin is generally preferred, since pig skin from abattoirs is readily available, and since the morphological and functional characteristics of pig skin are similar to that of human skin (**Meyer, W. et al., Curr. Probl. Dermatol. 7, 39-52, 1978**). However, presently pig skin models still have shortcomings: Handling and cultivation of completely functional ex vivo skin samples requires sophisticated handling techniques. Thus, such ex vivo models are generally considered cumbersome. In addition, the maximum time of viability of ex vivo skin samples is currently limited to seven days (cf. e.g. DE 10317400 A1). This maximum time of viability requires users to frequently obtain new skin samples, thereby reducing reproducibility, requiring frequent calibrations and, since the decline in viability can even affect short-term substance assays, requires a high precision and great care when analysing measured effects.

Current pig skin models generally fall into one of the following groups:
DE 19317400 A1 describes a method for manufacturing ex vivo pig skin samples, particularly for screening of wound healing and substance interactions with skin. Related thereto, the application describes a method for manufacturing male ex vivo pig skin. Pig skin samples are taken from the inner side of an arbitrary pig's ear. Fat tissue is cut off and the remaining epidermis and dermis are placed in a cultivation medium such that the epidermis is exposed to air. The publications do not provide any indication on the viability of the skin samples after seven days. Also, by removal of fat tissue significant parts of the hair bulb, apocrine and eccrine glands get lost. In addition, fat cells significantly influence the proliferation of dermal and epidermal cells **(Sugihara et al., Br. J**. **Dermatol. 144: 244-253, 2001; Misago et al., Br. J. Dermatol 139: 40-48,1998).** The model's prognostic value is therefore limited.
Rijnkels et al. **(Photochem., Photobiol., 2001, 73 (5): 499-504)** describe an ex vivo pig skin model comprising the use of a skin piece of 6 months old Yorkshire-Landsvarken domestic pigs. Skin pieces (5x50 mm) including the complete dermis (± 5 mm thickness) were tested for time and dose-related ultraviolet B damage. The skin samples were cultivated for a maximum of 6 days. Again, subcutaneous fat was removed. The model therefore has the same caveats as the aforementioned pig skin models. Further pig skin studies are summarized in the following Table 1:

**Table 1**

| Pig skin studies | | | |
|---|---|---|---|
| Part of pig skin, size | Life time | Application | References |
| Ear, Dermis + Epidermis, size: 0.25 cm² | Minimum 3 days | Screening of irritants Screening of irritants | Jacobs et al. (ATLA 28: 2779-292,2000) |
| Back skin, subcutaneous fat removed, subcutaneous fat removed, size: 2×3 cm | Not indicated | UV irradiation, Phtotoprotection, Photodegradation | Moison et al (Photochem. Photobiol 77:343-8, 2003) |
| Back skin, subcutaneous fat removed, size: 5x5 mm | Max. use for 144 h | Phototoxicity, Photoprotection | Rijnkels et al (Photochem. Photobiol. 73: 499-504,2001) |
| Part not indicated, fat scraped off, Size: 10x10 cm, | Not indicated | Photodegradation, Photosensitization | Sarabia et al. (J. Photochem. Photobiol. B 58: 32-8,2000) |
| Ears, full thickness organ, full size minus 5-10 cm distal to the cut. | Max. ca. 48 h | Screening of irritants and corrosives | Fentem et a. (Toxicol In Vitro. 15:57-93, 2001) |

It has also been tried to use ex vivo pig skin frozen in liquid nitrogen and stored at -70 °C until use. However, it has been repeatedly shown that there are differences in the results obtained with frozen skin compared to viable, fresh ex vivo skin (Sintov et al., Int. J. Pharm. 27: 55-62, 2006; Brain et al., Food. Chem. Toxicol. 43: 681-690, 2005; Kasting et al., **Pharm. Res.** 7: **1141-1146, 1990).**

There is thus a great need for a skin model for modelling a selected effect of a selected treatment of human skin. Preferably, the model should allow for skin sample viability of greater than 7 days and more preferably of greater than 10 days after exposition. Also, the model should allow assessing a huge variety of effects of human skin treatments. The model should be easy to use and should allow for a high prognostic value regarding the effects of a treatment of human skin.

### Description of the invention

A first object of the present invention is related to the use of a pig skin sample taken from a pigs' neck and/or dorsal part having a hair follicle density of at least 15 follicles per cm² for simulating or analyzing a treatment of human or animal skin, wherein the pig skin sample comprises an epidermis, dermis and subcutis layer.

The present invention, however, is also directed to a method for modelling, simulating or analyzing a selected effect of a selected treatment of human or animal skin, comprising the steps of:
(a) Providing a skin sample taken from a pigs' neck and/or dorsal part having a hair follicle density of at least 15 follicles per cm² of a pig, the sample including an epidermis, dermis and a subcutis layer;
(b) Subjecting the sample to the selected treatment; and
(c) Observing the effect of the treatment on the skin sample.

According to the invention, a pig skin sample is therefore used as a model for human or animal skin, when the pig skin sample comprises an epidermis, dermis and a subcutis ("fat") layer. Within the scope of the present invention, a skin sample is an isolated body of skin consisting of an epidermal layer, a dermal layer ("dermis", sometimes also termed "corium"), and a subcutis layer, preferably containing at least one complete/fully preserved and structurally intact hair bulb.

According to the invention, the skin sample comprises a cell layer of epidermis cells and corresponding tissues structure, preferably a stratum corneum section and a stratum basale section. Further, the skin sample comprises a dermal layer between the epidermis and the subcutis layer. The subcutis layer comprises cells of a pig subcutis, preferably adipose tissue cells, and preferably also has a subcutis tissue structure. The pig skin sample does not have to comprise a complete pig skin subcutis; it is sufficient that the pig skin sample comprises a subcutis layer with an average thickness of at least 0.5 mm, preferably at least 1 mm and even more preferred 1 to 3 mm.

So far, the beneficial effects of the presence of a subcutis layer for skin sample viability, reproducibility of skin sample test results and significance of skin sample test results, e.g. their value for predicting skin reactions of a human being subjected to the same treatment as the skin sample, had not been described or anticipated. Instead, the subcutis layer of prior art pig skin samples has been removed to achieve long term sample viability (cf. Rijnkels et al. and Jacobs et al.). As a matter of fact, according to WO 2004 092726 A1 (Universitätsklinikum Hamburg) it is explicitly preferred to scrub off the sub-cutis layer in order to facilitate absorption of nutrients. It has now been found that contrary to present day believe, the presence of a fat layer (subcutis layer) in pig skin samples beneficially increases sample viability, test reproducibility and prognostic value.

Surprisingly, it has now been found that sample viability is further correlated to hair follicle density. A skin sample having a hair follicle density of at least 15 (primary) follicles per cm² is highly viable and fully functional for more than seven days after obtaining the sample from a pig, and allows for the assessment of a whole range of different biological parameters in an easy manner, as is described in greater detail below.

Thus, it is particularly preferred that the skin sample of the present invention comprises at least one primary hair follicle, even more preferred at least two, three or more primary hair follicles. It is presently believed that skin sample viability is beneficially increased by interaction between hair follicle cells and respective surrounding cells of the pig skin sample.

Thus, it is particularly preferred that the pig skin sample comprises at least one, preferably at least two, three or more viable (primary) hair follicles. For the present invention, a hair follicle is deemed to be viable if it was able to produce a skin hair in the pig and is structurally intact, that is to say not disrupted or incomplete, in the skin sample. A hair follicle is deemed to be viable according to the present invention if the minimum distance between the hair bulb and the nearest edge of the skin sample is at least 1 mm and even more preferably 1 to 3 mm.

According to the invention, a skin sample is viable and completely functional as long as both of the following conditions are fulfilled:
(a) the skin sample contains at least 1.5 percent of skin cells stainable with the proliferation marker Ki-67, and
(b) the percentage of skin cells of the skin sample stainable with the proliferation marker Ki-67 is more than 25 percent compared to the skin cells stainable with the proliferation marker Ki-67 at the start of the incubation.

The effect of hair follicle density on pig skin sample viability had not been reported previously. It had been known that healing of porcine wounds is accelerated during anagen phase of hair follicle cycle (Stenn et al., **Physiol. Ref. 2001, 81: 479)** and that contact allergen sensitisation is suppressed in accordance with the hair follicle growth cycle. However, hair follicle impacts on skin have mostly been documented in rodents which in contrast to pigs and humans have a highly synchronized hair follicle cycle (Stenn et al., ibid.). Preferably, the skin sample has a hair follicle density of at least 20 hair follicles per cm², more preferably of 25 to 35 hair follicles per cm². Such skin samples have proven to be particularly long viable. They are thus easy to handle and do not require frequently obtaining new skin samples and new sample calibrations. Preferably, the skin sample is taken from the skin of a pig of up to 11 weeks, more preferably of 611 weeks and particularly preferably of an age of 8 to10 weeks. Such pigs generally weight up to 35 kg, preferably 15 to 35 kg and particularly preferred 23 to30 kg. It has now been found that the skin of such young pigs allows for considerably longer sample viability than a skin of older pigs.

According to the invention, pig skin samples are taken from a pig's neck and/or dorsal part. It has now been found that skin samples from these regions with the above indicated hair follicle density according to the invention have a ratio of anagen/catagen/telogen hair follicles which is closely similar to that of human scalp and have turned out to mimic human scalp reactions to treatments better than skin samples from other pig skins regions.

In preferred skin models of the present invention, the skin samples are of a domestic pig (Sus scorofa, sometimes also termed Sus domesticus), most preferably a Pietrain or a Landrace hybrid pig. Pietrain pig skin is particularly preferred since this race has a partly pigmented skin and thus allows the assessment of skin pigmentation effectors, i.e. tanning substances and skin whitening substances. The Landrace hybrid pigs do not have pigmented spots as the Pietrain hybrid has. However, the characteristics of the skin (with the exception of parameters that relate to pigmentation) closely resemble those of the Pietrain hybrid (particularly hair follicle density, skin thickness and hair follicle cycling). A Landrace hybrid pig skin sample can therefore be used as a (non-pigmented) control for Pietrain hybrid pig skin samples.

Preferably, the skin sample has a thickness of at least 2 mm, preferably of at least 3 mm and more preferred of 4 to 5 mm. In addition, the skin sample preferably has an epidermis surface area of at least 9 mm² and more preferred of 16 to 25 mm². Pig skin samples of these sizes have proven to be particularly viable. Thus, skin samples sizes with the following dimensions are preferred (length x width x thickness): (3-6) x (3-6) x (2-5) mm, preferably (4-5) x (4-5) x (3-4) mm. These sample sizes allow to completely include structurally intact, functioning hair follicles and hairs in the sample. Thus, it is particularly preferred for a skin sample with an epidermis surface area of 9 to 25 mm² to comprise at least three hair follicles, more preferably at least four hair follicles, and still more preferred at least five hair follicles.

Skin samples are preferably prepared from larger skin patches taken from a pig. Skin patches a taken preferably from living or slaughtered pigs, preferably the time between slaughtering and taking a skin patch is not longer than 1 hour. The area of the skin to be excised is preferably washed with distilled water and then dried, preferably with sterile gauzes. Afterwards, the hair shafts are cut be using an electric clipper at a length of 1 to 2 mm. Preferably, the skin is not injured during clipping. It is further preferred that all dorsal and/or neck skin where a skin patch is to be taken from is subsequently washed by using a surgical soap; the soap being removed from the skin by plugging with sterile gauzes soaked in distilled water. Afterwards, the skin is further cleaned with chlorhexidine and then dried with sterile gauzes. Lastly, the skin is restored by using a physiological saline solution.

The skin patches to be excised are carved with a scalpel in order to obtain lens-shaped patches. The patches preferably have a dimension of 5-8 cm (length) and 2-5 cm (width.), most preferably 6 cm x 4 cm. It is convenient to perform the excision by two cuts that follow the edges of the lens-shaped skin patch to be excised. The skin patch is then lifted carefully, e. g. by the use of forceps, and is gently detached from the muscle. The skin patch then includes epidermis, dermis and subcutis fat layer. The fat layer is preferably further reduced to a maximum thickness of 3 mm. It has been found that a fat layer of this thickness favourably allows nutrition of the tissue from culture media. Once excised, the skin patches are placed in a 50 ml tube containing 40 ml of transport medium and maintained at 4 °C until the patches arrives at the laboratory to be further processed. It has been found that this medium provides an effective protection of skin patches from bacterial contamination during the transfer from the abattoir to the laboratory, and it also protecting the pig skin patches tissue characteristics. A suitable transport medium is (Dulbecco Modified Eagle Medium (DMEM) with added penicillin (100 U/ml) and streptomycin (100 µg/ml). Most preferably, the excised skin patch is treated for not more than 4 hours, preferably not more than 3 hours with transport medium. Fast processing of the skin patches to obtain skin samples aids in ensuring minimal or no skin degeneration.

For preparing skin samples for the assessment of a selected effect of a skin treatment, skin patches are transferred to a culture medium. The culture medium preferably is free of fetal calf serum (FCS), and even more preferably is a complex culture medium like William's E medium. Of course, the culture medium composition is selected by the skilled person in view of the treatment and effect to assess. Thus, a generally less preferred culture medium maybe suitable under special circumstances. The culture medium can be supplemented by compounds known to improve the proliferation of skin and hair cells as e.g. vitamins or zinc salts. When preparing skin samples from skin patches, generally the edges of skin patches are cut and discarded. The remaining skin sample is placed on a sterile support, preferably cork, and cut to the desired sizes as given above, preferably to a size of 4x4x3 mm (length x width x thickness). Skin samples are preferably placed in a culture medium with the epidermis surfacing the air above the culture medium and the subcutaneous fat and dermis layers being completely immersed in the culture medium. It is however preferred to place skin samples on a sterile support like a cotton pad, will the support is soaked with culture medium, and the skin sample being oriented such that the fat and dermis layer are directed to the support and the epidermis being directed upwardly. Preferably, the sterile support and particularly the soaked cotton pad is replaced every 3 days. It is further preferred to place two or three skin samples on one support. The support is preferably located inside a well of a conventional-type six well culture plate. The skin samples can be used for excising/modelling a variety of treatments, among which are:
(a) effects of exposition of skin samples to substances, the substances being applied topically to the skin sample and/or systemically to the skin sample by mixing with the culture medium; or
(b) effects of skin sample irradiation.

Preferably, the skin samples are used for the assessment of one or more of the following effects, preferably caused by the application of a putatively effect-causing substance or other treatment:
- modulation of skin and/or hair pigmentation,
- modulation of hair growth,
- modulation of skin and/or hair viability and/or proliferation,
- modulation of fat metabolism,
- anti-cellulitis properties of substances,
- slimming,
- anti-aging effects, particularly by fat cell stimulation
- allergenic potential and/or irritation,
- UV protection, particularly UV erythema prevention, alleviation and healing,
- modulation of connecting tissue properties, particularly for the assessment of anti-wrinkle properties of substances,
- anti-oxidative effects,
- wound healing,
- modulation of skin barrier function,
- modulation of ion channels, especially neurofunctional channels and preferably channels activated by GABA, glutamate, acetylcholine, serotonin, adrenalin and ATP, and temperature sensitive channels (TRPM8, TRPV3, TRPV4, TRPV1, ANKTM, TRPV2),
- immunestimulation, immunesuppression,
- sebum stimulation, sebum suppression,
- anti-microbial effectiveness, particularly anti-acne effectiveness,
- sweat secretion decrease,
- substantivity of materials on a skin surface,
- film forming effectiveness,
- modulation of hair thickness,
- moisturization,
- phototoxicity,
- skin metabolism of compounds.
- [0039] The skin model of the invention is particularly suitable to assess the effects of
- flavour compounds, particularly allergenic potential assessment,
- fragrance compounds, particularly for substantivity and allergenic potential assessment,
- pharmaceutical compounds, particularly for skin penetration, skin damage alleviation and skin damage healing,
- insect repellents, particularly for skin penetration and skin damage,
- make up compounds, particularly pigments, particularly for skin penetration, skin damage or skin adhesiveness,
- hair care ingredients,
- nutraceuticals, dietary supplements or functional food.

The substances the effects of which are to be assessed by the skin model are applied topically by application to the epidermis, or systemically by addition to a cultivation medium. The substances can be applied in any form, including application as a pure substance, or a mixture with one or more other substances (that may or may not have effects on a skin model). The substance or substances can be applied as a solid, a gel, a cream or other multiphase composition, a liquid, foam or gas. The substance(s) and formulations to be tested (hereinafter: "formulation") on the pig skin model according to the invention preferably are or contain a total preferably of 0.01 weight percent to 30 weight percent, particularly preferably of 0.01 to 20 weight percent and very particularly preferably of 0.05 weight percent to 5 weight percent, based on the total weight of the formulation, of the mixture constituents
(a) ceramide/pseudoceramide,
(b) (alpha-)bisabolol and optionally
(c) cholesterol/phytosterols and
(d) fatty acids,
and can take the form of soap, syndet, liquid washing, shower and bath preparation, emulsion (as solution, dispersion, suspension, cream, lotion or milk, depending on preparative method and ingredients, of the type W/O, O/W or multiple emulsion, PIT emulsion, emulsion foam, microemulsion, nanoemulsion, Pickering emulsion), ointment, paste, gel (including hydrogel, hydrodispersion gel, oleogel), oil, toner, balsam, serum, powder, eau de toilette, eau de Cologne, perfume, wax, stick, roll-on, (pump) spray, aerosol (foaming, non-foaming or after-foaming), foot care product (including keratolytics, deodorant), pre-shave or after-shave (balm, lotion), depilatory product, hair care product, e.g. shampoo (incl. 2-in-1 shampoo), conditioner, hair treatment, hair tonic, hair rinse, hair cream, pomade, perming and fixing product, hair straightening product (defrizzer, relaxer), hair strengthener, styling aid (e.g. gel or wax), bleach, hair dye (e.g. temporary, direct, semipermanent, permanent hair dye), nail care product, e.g. nail varnish and nail varnish remover, deodorant and/or antiperspirant, mouthwash, make-up, make-up remover or decorative cosmetic (e.g. powder, eye shadow, kajal stick, lipstick).

### Cosmetic auxiliary agents

It can be advantageous to provide the formulations to be applied to the pig skin model according to the invention in encapsulated form, e.g. in gelatin, wax materials, liposomes, cellulose capsules or cyclodextrin capsules.

Other conventional cosmetic auxiliary substances and additives can be present in formulations in amounts advantageously of 5 - 99 weight percent, preferably of 10 - 80 weight percent, based on the total weight of the mixture. The formulations can also contain water in an amount of up to 99.99 weight percent, preferably of 5 - 80 weight percent, based on the total weight of the formulation.

The formulations can be or contain cosmetic auxiliary substances and additives such as those conventionally used in cosmetic preparations, e.g. sunscreens, preservatives, bactericides, fungicides, virucides, cooling substances, insect repellents (e.g. DEET, IR 3225, Dragorepel), plant extracts, antiinflammatory substances, wound healing accelerators (e.g. chitin or chitosan and its derivatives), film-forming substances (e.g. polyvinylpyrrolidones or chitosan or its derivatives), customary antioxidants, vitamins (e.g. vitamin C and derivatives, tocopherols and derivatives, vitamin A and derivatives), 2-hydroxycarboxylic acids (e.g. citric acid, malic acid, L-, D- or DL-lactic acid), skin colourants (e.g. walnut extracts or dihydroxyacetone), active ingredients for promoting hair growth (e.g. minoxidil, diphencyprone, hormones, finasteride, phytosterols such as beta-sitosterol, biotin, or extracts of Cimicifuga racemosa, Eugenia caryophyllata or Hibiscus rosasinensis, barley, hops, or rice or wheat hydrolysates), skin care products (e.g. cholesterol, ceramides, pseudoceramides), softening, moisturizing and/or moisture-retaining substances (e.g. glycerol or urea), fats, oils, saturated fatty acids, monounsaturated or polyunsaturated fatty acids, alpha-hydroxy acids, polyhydroxy fatty acids or their derivatives (e.g. linoleic acid, alpha-linolenic acid, gamma-linolenic acid or arachidonic acid and their respective natural or synthetic esters), waxes or other conventional constituents of a cosmetic or dermatological formulation, such as alcohols, polyols, polymers, foam stabilizers, electrolytes, organic solvents, silicone derivatives or chelating agents (e.g. ethylenediaminetetraacetic acid and derivatives), antidandruff substances (e.g. climbazole, ketoconazole, piroctonoleamine, zinc pyrithione), hair care products, perfumes, antifoams, dyestuffs, pigments with a colouring action, thickeners (advantageously silicon dioxide, aluminium silicates such as bentonites, polysaccharides or their derivatives, e.g. hyaluronic acid, guar kernel flour, xanthan gum, hydroxypropyl methyl cellulose or allulose derivatives, particularly advantageously polyacrylates such as carbopols, or polyurethanes), surface-active substances, emulsifiers, plant parts and plant extracts (e.g. arnica, aloe, beard lichen, ivy, stinging nettle, ginseng, henna, camomile, marigold, rosemary, sage, horsetail or thyme), animal extracts, e.g. royal jelly or propolis, proteins, protein hydrolysates, yeast extracts, hop and wheat extracts, peptides or thymus extracts.

The amounts of cosmetic or dermatological auxiliary substances and additives and perfume to be used can readily be determined by those skilled in the art on a simple trial-and-error basis, as a function of the particular type of product.

### UV filters

Advantageously the formulations be or contain at least one UVA filter and/or at least one UVB filter and/or at least one inorganic pigment. The mixtures can take a variety of forms, e.g. those conventionally used for sunscreen preparations for protecting the skin and hair from ultraviolet radiation. They can thus form e.g. a solution, an emulsion of the water-in-oil (W/O) type or oil-in-water (O/W) type, or a multiple emulsion, for example of the water-in-oil-in-water (W/O/W) type, a gel, a hydrodispersion, a solid stick or else an aerosol. The total amount of filter substances is from 0.01 weight percent to 40 weight percent, preferably from 0.1 percent to 10 weight percent and particularly preferably from 1.0 to 5.0 weight percent, based on the total weight of the mixture, in order to provide cosmetic mixtures (preparations).

Examples of advantageous UV filters are:
- p-aminobenzoic acid
- ethyl p-aminobenzoate, ethoxylated (25 mol)
- 2-ethylhexyl p-dimethylaminobenzoate
- ethyl p-aminobenzoate, N-propoxylated (2 mol)
- glyceryl p-aminobenzoate
- homomenthyl salicylate (homosalate) (Neo Heliopan^{(R)}HMS)
- 2-ethylhexyl salicylate (Neo Heliopan^{(R)}OS)
- triethanolamine salicylate
- 4-isopropylbenzyl salicylate
- menthyl anthranilate (Neo Heliopan^{(R)}MA)
- ethyl diisopropylcinnamate
- 2-ethylhexyl p-methoxycinnamate (Neo Heliopan^{(R)}AV)
- methyl diisopropylcinnamate
- isoamyl p-methoxycinnamate (Neo Heliopan^{(R)}E 1000)
- p-methoxycinnamic acid diethanolamine salt
- isopropyl p-methoxycinnamate
- 2-ethylhexyl 2-cyano-3,3-diphenylacrylate (Neo Heliopan^{(R)}303)
- ethyl 2-cyano-3,3'-diphenylacrylate
- 2-phenylbenzimidazolesulfonic acid and salts (Neo Hellopan^{(R)}Hydro)
- 3-(4'-trimethylammonium)benzylidenebornan-2-one methylsulfate
- terephthalylidenedibornanesulfonic acid and salts (Mexoryl^{(R)}SX)
- 4-t-butyl-4'-methoxydibenzoylmethane (avobenzone)/(Neo Heliopan^{(R)}357)
- beta-imidazol-4(5)-acrylic acid (urocanic acid)
- 2-hydroxy-4-methoxybenzophenone (Neo Heliopan^{(R)}BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octyloxybenzophenone
- 2-hydroxy-4-methoxy-4'-methylbenzophenone
- 3-(4'-sulfo)benzylidenebornan-2-one and salts
- 3-(4'-methylbenzylidene)-d,l-camphor (Neo Heliopan^{(R)}MBC)
- 3-benzylidene-d,l-camphor
- 4-isopropyldibenzoylmethane
- 2,4,6-trianilino(p-carbo-2'-ethylhexyl-1'-oxy)-1,3,5-triazine
- phenylenebisbenzimidazyltetrasulfonic acid disodium salt (Neo Heliopan^{(R)}AP)
- 2,2'-(1,4-phenylene)bis(1H-benzimidazole-4,6-disulfonic acid) monosodium salt
- N-[(2 and 4)-[2-(oxoborn-3-ylidene)methyl]benzyl]acrylamide polymer
- phenol,2-(2H-benzotriazol-2-yl)-4-methyl-6-(2-methyl-3-(1,3,3,3-tetramethyl-1-((trimethylsilyl)oxy)disiloxanyl)propyl) (Mexoryl^{(R)}XL)
- 4,4'-[(6-[4-(1,1-dimethyl)aminocarbonyl)phenylamino]-1,3,5-triazine-2,4-diyldiimino]bis(benzoic acid 2-ethylhexyl ester) (Uvasorb^{(R)}HEB)
- 2,2'-methylenebis(6-(2H-benztriazol-2-yl)-4-(1,1,3,3-tetramethylbutyl)phenol) (Tinosorb^{(R)}M)
- 2,4-bis[4-(2-ethylhexyloxy)-2-hydroxyphenyl]-1,3,5-triazine
- benzylidene malonate polysiloxane (Parsol^{(R)}SLX)
- glyceryl ethylhexanoate dimethoxycinnamate
- disodium 2,2'-dihydroxy-4,4'-dimethoxy-5,5'-disulfobenzophenone
- dipropylene glycol salicylate
- sodium hydroxymethoxybenzophenonesulfonate
- 4,4',4-(1,3,5-triazine-2,4,6-triyltriimino)tris(benzoic acid 2-ethylhexyl ester) (Uvinul^{(R)}T150)
- 2,4-bis[{4-(2-ethylhexyloxy)-2-hydroxy}phenyl]-6-(4-methoxyphenyl)-1,3,5-triazine (Tinosorb^{(R)}S)
- 2,4-bis[{(4-(3-sulfonato)-2-hydroxypropoxy)-2-hydroxy}phenyl]-6-(4-methoxyphenyl)-1,3,5-triazine sodium salt
- 2,4-bis[{(3-(2-propoxy)-2-hydroxypropoxy)-2-hydroxy}phenyl]-6-(4-methoxyphenyl)-1,3,5-triazine
- 2,4-bis[{4-(2-ethylhexyloxy)-2-hydroxy}phenyl]-6-[4-(2-methoxyethylcarbonyl)phenylamino]-1,3,5-triazine
- 2,4-bis[{4-(3-(2-propoxy)-2-hydroxypropoxy)-2-hydroxy}phenyl]-6-[4-(2-ethylcarboxy)phenylamino]-1,3,5-triazine
- 2,4-bis[{4-(2-ethylhexyloxy)-2-hydroxy}phenyl]-6-(1-methylpyrrol-2-yl)-1,3,5-triazine
- 2,4-bis[{4-tris(trimethylsiloxysilylpropoxy)-2-hydroxy}phenyl]-6-(4-methoxyphenyl)-1,3,5-triazine
- 2,4-bis[{4-(2"-methylpropenyloxy)-2-hydroxy}phenyl]-6-(4-methoxyphenyl)-1,3,5-triazine
- 2,4-bis[{4-(1',1',3',5',5',S'-heptamethylsiloxy-2"-methylpropoxy)-2-hydroxy}phenyl]-6-(4-methoxyphenyl)-1,3,5-triazine
- hexyl 2-(4-diethylamino-2-hydroxybenzoyl)benzoate (Uvinul^{(R)} A Plus)
- indanylidene compounds according to DE 100 55 940 (= WO 02/38537)
- [0048] The following UV absorbers are particularly suitable for combination:
- p-aminobenzoic acid
- 3-(4'-trimethylammonium)benzylidenebornan-2-one methylsulfate
- homomenthyl salicylate (Neo Heliopan^{(R)}HMS)
- 2-hydroxy-4-methoxybenzophenone (Neo Heliopan^{(R)}BB)
- 2-phenylbenzimidazolesulfonic acid (Neo Heliopan^{(R)}Hydro)
- terephthalylidenedibornanesulfonic acid and salts (Mexoryl^{(R)}SX)
- 4-tert-butyl-4'-methoxydibenzoylmethane (Neo Heliopan^{(R)}357)
- 3-(4'-sulfo)benzylidenebornan-2-one and salts
- 2-ethylhexyl 2-cyano-3,3-diphenylacrylate (Neo Heliopan^{(R)}303)
- N-[(2 and 4)-[2-(oxoborn-3-ylidene)methyl]benzyl]acrylamide polymer
- 2-ethylhexyl p-methoxycinnamate (Neo Heliopan^{(R)}AV)
- ethyl p-aminobenzoate, ethoxylated (25 mol)
- isoamyl p-methoxycinnamate (Neo Heliopan^{(R)}E1000)
- 2,4,6-trianilino(p-carbo-2'-ethylhexyl-1'-oxy)-1,3,5-triazine (Uvinu^{(R)}T150)
- phenol,2-(2H-benzotriazol-2-yl)-4-methyl-6-(2-methyl-3-(1,3,3,3-tetramethyl-1-((trimethylsilyl)oxy)disiloxanyl)-propyl) (Mexoryl^{(R)}XL)
- 4,4'-[(6-[4-(1,1-dimethyl)aminocarbonyl)phenylamino]-1,3,5-triazine-2,4-diyldiimino]bis(benzoic acid 2-ethylhexyl ester) (Uvasorb^{(R)}HEB)
- 3-(4'-methylbenzylidene)-d,l-camphor (Neo Helipan^{(R)}MBC)
- 3-benzylidenecamphor
- 2-ethylhexyl salicylate (Neo Helipan^{(R)}OS)
- 2-ethylhexyl 4-dimethylaminobenzoate (Padimate O)
- hydroxy-4-methoxybenzophenone-5-sulfonic acid and Na salt
- 2,2'-methylenebis(6-(2H-benztriazol-2-yl)-4-(1,1,3,3-tetramethylbutyl)phenol) (Tinosorb^{(R)}M)
- phenylenebisbenzimidazyltetrasulfonic acid disodium salt (Neo Heliopan^{(R)}AP)
- 2,4-bis[{4-(2-ethylhexyloxy)-2-hydroxy}phenyl]-6-(4-methoxyphenyl)-1,3,5-triazine (Tinosorb^{(R)}S)
- benzylidene malonate polysiloxane (Parso^{(R)}SLX)
- menthyl anthranilate (Neo Heliopan^{(R)}MA)
- hexyl 2-(4-diethylamino-2-hydroxybenzoyl)benzoate (Uvinul^{(R)} A Plus)
- indanylidene compounds according to DE 100 55 940 (= WO 2002 38537 A1)

Advantageous inorganic light-protecting pigments are finely disperse metal oxides and metal salts, for example titanium dioxides, zinc oxide (ZnO), iron oxides (e.g. Fe₂O₃), aluminium oxide (Al₂O₃), cerium oxides (e.g. Ce₂O₃), manganese oxides (e.g. MnO), zirconium oxide (ZrO₂), silicon oxide (SiO₂), mixed oxides of the corresponding metals, and mixtures of such oxides, barium sulfate and zinc stearate. Particularly preferred pigments are those based on TiO₂ or zinc oxide. In preferred embodiments the particles have a mean diameter of less than 100 nm, preferably of between 5 and 50 nm and particularly preferably of between 15 and 30 nm. They can have a spherical shape, but it is also possible to use particles with an ellipsoid shape or a shape that differs from spherical in some other way. The pigments can also be surface-treated, i.e. hydrophilized or hydrophobized. Typical examples are coated titanium dioxides, e.g. titanium dioxide T 805 (Degussa) or Eusolex^{(R)} T2000 (Merck), or coated zinc oxide, e.g. zinc oxide NDM, suitable hydrophobic coating agents being primarily silicones and especially trialkoxyoctylsilanes or simethicones. So-called micro-pigments or nanopigments are preferably used in sunscreen products, zinc micro-pigments or nanopigments being particularly preferred.

The total amount of inorganic pigments, especially hydrophobic inorganic micro-pigments, in the finished cosmetic or dermatological formulations advantageously ranges from 0.1 to 30 weight percent, preferably from 0.1 to 10.0 and particularly preferably from 0.5 to 6.0 weight percent, based on the total weight of the formulations.

### Anti irritants and anti-inflammatories

Other anti-irritants apart from (alpha-)bisabolol can also be used in or as formulations to be tested with the aid of the pig skin model according to the invention. Anti-irritants can be any anti-inflammatory or redness-alleviating and antipruritic substances that are suitable or customary for cosmetic and/or dermatological applications. Preferred anti-inflammatory or redness-alleviating and antipruritic substances (anti-irritants) are steroidal anti-inflammatory substances of the corticosteroid type, e.g. hydrocortisone, dexamethasone, dexamethasone phosphate, methylprednisolone or cortisone, it being possible to extend the list by adding other steroidal anti-inflammatories. Non-steroidal anti-inflammatories can also be used. The following may be mentioned as examples: oxicams such as piroxicam or tenoxicam; salicylates such as aspirin, Disalcid, Solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen or benoxaprofen; or pyrazoles such as phenylbutazone, oxyphenylbutazone, febrazone or azapropazone. Natural anti-inflammatory or redness-alleviating and antipruritic substances can be used as alternatives, possibilities being plant extracts, special potent plant extract fractions, and high-purity active substances isolated from plant extracts. Particular preference is given to extracts, fractions and active substances from camomile, Aloe vera, Commiphora species, Rubia species, Echinacea species, willows, willow herb, oats, black and green tea, gingko, coffee, pepper, redcurrant/blackcurrant, tomato, vanilla and almonds, as well as pure substances such as, inter alia, apigenin-7-glucoside, boswellic acid, phytosterols, glycyrrhizinic acid, glabridin or licochalcone A.

In terms of the invention, particular preference is given to panthenol, boswellic acid and extracts and isolated high-purity active substances from oats (e.g. avenanthramides) and Echinacea, and mixtures thereof.

### Antioxidants

The formulations can also be or contain antioxidants, it being possible to use any antioxidants suitable or customary for cosmetic and/or dermatological applications. The antioxidants are advantageously selected from the group comprising amino acids (e.g. glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. alpha-carotene, beta-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and their glycosyl, N-acetyl, methyl, ethyl, propyl, amyl, butyl, lauryl, palmitoyl, oleyl, gamma-linoleyl, cholesteryl and glyceryl esters) and their salts, dilauryl thiodipropionate, distearyl thiodipropionate, thiodipropionic acid and derivatives thereof (esters, ethers, peptides, lipids, nucleotides, nucleosides and salts), sulfoximine compounds (e.g. buthionine sulfoximine, homocysteine sulfoximine, buthionine sulfone, penta-, hexa-, heptathionine sulfoximine) in very small tolerable doses, (metal) chelators, e.g. alpha-hydroxy fatty acids, palmitic acid, phytic acid, lactoferrin, alpha-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. gamma-linolenic acid, linoleic acid, oleic acid), folic acid and derivatives thereof, ubiquinone and ubiquinol and derivatives thereof, vitamin C and derivatives (e.g. ascorbyl palmitate, Mg ascorbylphosphate, ascorbyl acetate, ascorbyl glycosides such as 6-O-acyl-2-O-alpha-D-glucopyranosyl-L-ascorbic acid, 6-O-acyl-2-O-beta-D-glucopyranosyl-L-ascorbic acid, 2-O-alpha-D-glucopyranosyl-L-ascorbic acid or 2-O-beta-D-glucopyranosyl-L-ascorbic acid), tocopherols and derivatives thereof (e.g. vitamin E acetate), vitamin A and derivatives thereof (vitamin A palmitate), coniferyl benzoate from benzoin, rutic acid and derivatives thereof, alpha-glycosylrutin, quercetin and derivatives thereof, rosmaric acid, carnosol, carnosolic acid, resveratrol, caffeic acid and derivatives thereof, sinapic acid and derivatives thereof, ferulic acid and derivatives thereof, furfurylideneglucitol, curcuminoids, butylhydroxytoluene, butylhydroxyanisole, nordihydroguaiacic acid, nordihydroguaiaretic acid, trihydroxybutyrophenone, uric acid and derivatives thereof, mannose and derivatives thereof, superoxide dismutase, zinc and derivatives thereof (e.g. ZnO, ZnSO₄), selenium and derivatives thereof (e.g. selenium methionine), stilbenes and derivatives thereof (e.g. stilbene oxide, transstilbene oxide), derivatives (salts, esters, ethers, sugars, nucleotides, nucleosides, peptides and lipids) of said active substances, or antioxidative extracts or fractions of plants such as green tea, rooibos, honeybush, grape, rosemary, sage, balm, thyme, lavender, olive, oats, cacao, gingko, ginseng, liquorice, honeysuckle, Sophora, Pueraria, Pinus, Citrus, Phyllanthus emblica or St John's wort.

The amount of antioxidants (one or more compounds) in the formulations is preferably 0.01 to 20 weight percent, particularly preferably 0.05 - 10 weight percent and very particularly preferably 0.2 - 5 weight percent, based on the total weight of the preparation.

If vitamin E and/or its derivatives represent the antioxidant(s), their respective concentrations are advantageously chosen from the range between 0.001 and 10 weight percent, based on the total weight of the formulation.

If vitamin A or vitamin A derivatives, or carotenes or their derivatives, represent the antioxidant(s), their respective concentrations are advantageously chosen from the range between 0.001 and 10 weight percent, based on the total weight of the formulation.

### Active substances

The (cosmetic) formulations can also be or contain active substances and active substance combinations for combating skin ageing and wrinkling. It is possible here, according to the invention, to use any active substances for combating skin ageing and wrinkling that are suitable or customary for cosmetic and/or dermatological applications. In this respect, advantageous active substances for combating skin ageing and wrinkling are soya protein or protein hydrolysates, soya isoflavones, hydrolysed rice protein, hydrolysed hazelnut protein, oligopeptides from hydrolysed Hibiscus esculentus extract, wheat protein, beta-glucans, e.g. from oats, and derivatives thereof, glycoproteins, ursolic acid and its salts, betulin, betulinic acid and its salts, retinol, retinol palmitate, propyl gallate, precocenene, 6-hydroxy-7-methoxy-2,2-dimethyl-1(2H)-benzopyran, 3,4-dihydro-6-hydroxy-7-methoxy-2,2-dimethyl-1(2H)-benzopyran, creatine, or other synthetic or natural active substances for combating skin ageing and wrinkling, it also being possible for the latter to be used in the form of an extract of plants such as green tea, Rubus fruticosus, Sanguisorba officinalis, Centella asiatica, Ribes nigrum, Passiflora incarnata, Phyllanthus emblica, okra, algae, evening primrose, rosemary, sage, Echinacea, birch, apple or soya.
beta-Glucan is particularly preferably used as another active substance for combating skin ageing; 1,3-1,4-linked beta-glucan from oats, Rubus fruticosus extract or wheat protein is very particularly preferred.

Formulations in the form of a cosmetic preparation can advantageously also be or contain moisturizers. The following substances are examples of moisturizers used: sodium lactate, urea and urea derivatives, alcohols, glycerol, diols such as propylene glycol, 1,2-pentanediol, 1,2-hexanediol and 1,2-octanediol, collagen, elastin or hyaluronic acid, diacyl adipates, petrolatum, urocanic acid, lecithin, panthenol, phytantriol, lycopene, (pseudo)ceramides, glyco-sphingolipids, cholesterol, phytosterols, chitosan, chondroitin sulfate, lanolin, lanolin esters, amino acids, alpha-hydroxy acids (e.g. citric acid, lactic acid, malic acid) and derivatives thereof, mono-, di- and oligosaccharides such as glucose, galactose, fructose, mannose, fruit sugar and lactose, polysugars such as beta-glucans, especially 1,3-1,4-beta-glucan from oats, alpha-hydroxy fatty acids, triterpene acids such as betulinic acid or ursolic acid, and algae extracts.

The formulations can also be or used together with osmolytes. The following may be mentioned as examples of osmolytes: substances from the group comprising sugar alcohols (myoinositol, mannitol, sorbitol), quaternary amines such as taurine, choline, betaine, betaine glycine and ectoine, diglyceryl phosphate, phosphorylcholine, glycerophosphorylcholine, amino acids such as glutamine, glycine, alanine, glutamate, aspartate or proline, phosphatidylcholine, phosphatidylinositol, inorganic phosphates, and polymers of said compounds such as proteins, peptides, polyamino acids and polyols. All osmolytes have a skinmoisturizing effect at the same time.

Preferably, formulations can also be or contain active substances which stimulate skin and hair tinting or bronzing in a chemical or natural way, thereby achieving a more rapid action based on synergistic effects. Particularly preferably, said substances are substrates or substrate analogues of tyrosinase, such as L-tyrosine, L-DOPA or L-dihydroxyphenylalanine, stimulators of tyrosinase activity or expression, such as theophylline, caffeine, proopiomelanocortin peptides such as ACTH, alpha-MSH, their peptide analogues and other substances that bind to the melanocortin receptor, peptides such as Val-Gly-Val-Ala-Pro-Gly, Lys-Ile-Gly-Arg-Lys or Leu-Ile-Gly-Lys, purines, pyrimidines, folic acid, copper salts such as copper gluconate, chloride or pyrrolidonate, flavonoids, flavanone glycosides such as naringin and hesperidin, 1,3,4-oxadiazole-2-thiols such as 5-pyrazin-2-yl-1,3,4-oxadiazole-2-thiol, melanin derivatives such as Melasyn-100 and MelanZe, diacylglycerols, aliphatic or cyclic diols, psoralenes, prostaglandins and their analogues, adenylate cyclase activators, and compounds which activate the transfer of melanosomes into keratinocytes, such as serine proteases or PAR-2 receptor agonists, extracts of plants and plant parts of Chrysanthemum species and Sanguisorba species, walnut extracts, urucum extracts, rhubarb extracts, erytrulose and dihydroxyacetone.

The formulations can advantageously be or be used in combination with skin-lightening substances. Any skin-lightening substances that are conventional or customary for cosmetic and/or dermatological applications can be used according to the invention. Advantageous skin-lightening substances in this respect are koji acid (5-hydroxy-2-hydroxymethyl-4-pyranone), koji acid derivatives, e.g. koji acid dipalmitate, arbutin, ascorbic acid, ascorbic acid derivatives, hydroquinone, hydroquinone derivatives, resorcinol, sulfur-containing molecules, e.g. glutathione or cysteine, alpha-hydroxy acids (e.g. citric acid, lactic acid, malic acid) and derivatives thereof, N-acetyltyrosine and derivatives, undecenoylphenylalanine, gluconic acid, 4-alkylresorcinols, chromone derivatives such as aloesin, flavonoids, thymol derivatives, 1-aminoethylphosphinic acid, thiourea derivatives, ellagic acid, nicotinamide, zinc salts such as zinc chloride or gluconate, thujaplicin and derivatives, triterpenes such as maslinic acid, sterols such as ergosterol, benzofuranones such as senkyunolide, vinyl- and ethylguaiacol, inhibitors of nitrogen oxide synthesis, e.g. L-nitroarginine and derivatives thereof, 2,7-dinitroindazole or thiocitrulline, metal chelators (e.g. alpha-hydroxy fatty acids, palmitic acid, phytic acid, lactoferrin, humic acid, bile acid, bile extracts, bilirubin, biliverdin, EDTA, EGTA and derivatives thereof), retinoids, soya milk, serine protease inhibitors, lipoic acid or other synthetic or natural active substances for lightening the skin and hair, the latter also being used in the form of plant extracts, e.g. bearberry extract, rice extract, liquorice root extract or constituents obtained therefrom by enrichment, such as glabridin or licochalcone A, Artocarpus extract, extract of Rumex and Ramulus species, extracts of pine species (Pinus) and extracts of Vitis species or stilbene derivatives obtained therefrom by enrichment, and extracts of Saxifraga, mulberry, Scutelleria and/or grapes.

### Surfactants

Formulations in the form of cosmetic preparations can also contain anionic, cationic, non-ionic and/or amphoteric surfactants, especially if crystalline or microcrystalline solids, for example inorganic micro-pigments, are to be incorporated into the mixtures.

Anionic surfactants normally have carboxylate, sulfate or sulfonate groups as functional groups. In aqueous solution they form negatively charged organic ions in an acidic or neutral medium. Cationic surfactants are almost exclusively characterized by the presence of a quaternary ammonium group. In aqueous solution they form positively charged organic ions in an acidic or neutral medium. Amphoteric surfactants contain both anionic and cationic groups and accordingly behave as anionic or cationic surfactants in aqueous solution, depending on the pH. They have a positive charge in a strongly acidic medium and a negative charge in an alkaline medium. In the neutral pH range, on the other hand, they are zwitterionic. Non-ionic surfactants typically have polyether chains and do not form ions in an aqueous medium.

### a) Anionic surfactants

### Anionic surfactants that can advantageously be used are acylamino acids (and their salts) such as:

- acylglutamates, for example sodium acylglutamate, di-TEA palmitoylaspartate and sodium caprylic/capric glutamate,
- acylpeptides, for example palmitoyl-hydrolysed milk protein, sodium cocoyl-hydrolysed soya protein and sodium/ potassium cocoyl-hydrolysed collagen,
- sarcosinates, for example myristoyl sarcosine, TEA lauroylsarcosinate, sodium lauroylsarcosinate and sodium cocoylsarcosinate,
- taurates, for example sodium lauroyltaurate and sodium methylcocoyltaurate,
- acyllactylates, lauroyllactylate, caproyllactylate, stearoyllactylate,
- alaninates,carboxylic acids and derivatives, such as:
- lauric acid, aluminium stearate, magnesium alkanolate and zinc undecylenate,
- carboxylic acid esters, for example calcium stearoyllactylate, laureth-6 citrate and sodium PEG-4 lauramidecarboxylate,
- carboxylic acid ethers, for example sodium laureth-13 carboxylate and sodium PEG-6 cocamidecarboxylate,
- phosphoric acid esters and salts, such as DEA oleth-10 phosphate and dilaureth-4 phosphate,sulfonic acids and salts, such as:
- acylisethionates, e.g. sodium/ammonium cocoylisethionate,
- alkylarylsulfonates,
- alkylsulfonates, for example sodium cocomonoglyceridesulfate, sodium C₁₂₋₁₄-olefinsulfonate, sodium laurylsulfoacetate and magnesium PEG-3 cocamidesulfate,
- sulfosuccinates, for example sodium dioctylsulfosuccinate, disodium laureth sulfosuccinate, disodium laurylsulfosuccinate and disodium undecylenamido MEA sulfosuccinate,and sulfuric acid esters such as:
- alkyl ether sulfate, for example sodium, ammonium, magnesium, MIPA and TIPA laureth sulfate, sodium myreth sulfate and sodium C12-13 pareth sulfate,
- alkylsulfates, for example sodium, ammonium and TEA laurylsulfate.

### b) Cationic surfactants

The following cationic surfactants can advantageously be used:
- alkylamines,
- alkylimidazoles,
- ethoxylated amines and
- quaternary surfactants: RNH₂CH₂CH₂COO⁻ (at pH = 7) RNHCH₂CH₂COO- B⁺ (at pH = 12), B⁺ = any cation, e.g. Na⁺
- esterquats

Quaternary surfactants contain at least one N atom covalently bonded to 4 alkyl or aryl groups. This results in a positive charge, independently of the pH. Alkylbetaine, alkylamido-propylbetaine and alkylamidopropylhydroxysulfaine are advantageous. The cationic surfactants used can also preferably be selected from the group comprising quaternary ammonium compounds, especially benzyltrialkylammonium chlorides or bromides, for example benzyldimethylstearylammonium chloride, alkyltrialkylammonium salts, for example cetyltrimethylammonium chloride or bromide, alkyldimethylhydroxyethylammonium chlorides or bromides, dialkyldimethylammonium chlorides or bromides, alkylamidoethyltrimethylammonium ether sulfates, alkylpyrimidinium salts, for example laurylpyrimidinium or cetylpyridinium chloride, imidazoline derivatives, and compounds of cationic character, such as amine oxides, for example alkyldimethylamine oxides or alkylaminoethyldimethylamine oxides. Cetyltrimethylammonium salts can be used to particular advantage.

### c) Amphoteric surfactants

The following amphoteric surfactants can advantageously be used:
- acyl/dialkylethylenediamine, for example sodium acylamphoacetate, disodium acylamphodipropionate, disodium alkylamphodiacetate, sodium acylamphohydroxypropylsulfonate, disodium acylamphodiacetate and sodium acylamphopropionate,
- N-alkylamino acids, for example aminopropylalkylglutamide, alkylaminopropionic acid, sodium alkylimidodipropionate and lauroamphocarboxyglycinate.
- D. Non-ionic surfactants
- [0069] The following non-ionic surfactants can advantageously be used:
- alcohols,
- alkanolamides such as cocamides MEA/DEA/MIPA,
- amine oxides such as cocamidopropylamine oxide,
- esters formed by the esterification of carboxylic acids with ethylene oxide, glycerol, sorbitan or other alcohols,
- ethers, for example ethoxylated/propoxylated alcohols,ethoxylated/propoxylated esters, ethoxylated/propoxylated glycerol esters, ethoxylated/propoxylated cholesterols, ethoxylated/propoxylated triglyceride esters, ethoxylated/ propoxylated lanolin, ethoxylated/propoxylated polysiloxanes, propoxylated POE ethers, and alkylpolyglycosides such as laurylglucoside, decylglycoside and cocoglycoside,
- sucrose esters and ethers,
- polyglycerol esters, diglycerol esters and monoglycerol esters,
- methylglucose esters, hydroxy acid esters.

It is also advantageous to use a combination of anionic and/or amphoteric surfactants with one or more non-ionic surfactants.

The surface-active substance can be present in a concentration of between 1 and 98 weight percent in the mixtures to be used according to the invention, based on the total weight of the mixture.

### Lipid phase additives

A lipid phase in formulations used in the pig skin model according to the invention can advantageously be selected from the following groups of substances:
- mineral oils (advantageously paraffin oil), mineral waxes,
- fatty oils, fats, waxes and other natural and synthetic fatty substances, preferably esters of fatty acids with alcohols of low C number, e.g. with isopropanol, propylene glycol or glycerol, or esters of fatty alcohols with alkanoic acids of low C number or with fatty acids,
- alkyl benzoates,
- silicone oils such as dimethylpolysiloxanes, diethylpolysiloxanes, diphenylpolysiloxanes and mixed forms thereof,
- hydrocarbons (advantageously squalane or squalene),
- synthetic or semisynthetic triglyceride oils (e.g. triglycerides of capric or caprylic acid),
- natural oils (one or more nurturing animal and/or vegetable fats and oils, such as olive oil, sunflower oil, refined soya oil, palm oil, sesame oil, rapeseed oil, almond oil, borage oil, evening primrose oil, coconut oil, shea butter, jojoba oil, oat oil, sperm oil, tallow, neatsfoot oil and lard),and optionally other nurturing constituents, for example fatty alcohols having 8-30 C atoms, it being possible for the latter to be saturated or unsaturated and linear or branched. Examples of fatty alcohols which can be used are decanol, decenol, octanol, octenol, dodecanol, dodecenol, octadienol, decadienol, dodecadienol, oleyl alcohol, ricinoleyl alcohol (9-cis-octadecene-1,12-diol), erucyl alcohol, stearyl alcohol, isostearyl alcohol, cetyl alcohol, lauryl alcohol, myristyl alcohol, arachidyl alcohol, caprylic alcohol, capric alcohol, linoleyl alcohol, linolenyl alcohol and behenyl alcohol, and their Guerbet alcohols, the list being extendable almost without limit by other alcohols of chemically related structure. The fatty alcohols preferably originate from natural fatty acids and are conventionally prepared from the corresponding fatty acid esters by reduction. It is also possible to use fatty alcohol fractions formed by reduction from naturally occurring fats and fatty oils, e.g. tallow, groundnut oil, colza oil, cottonseed oil, soya oil, sunflower oil, palm kernel oil, linseed oil, maize oil, castor oil, rapeseed oil, sesame oil, cacao butter and coconut fat. Synthetic ester oils may also be present. Preferred esters are those of saturated and/or unsaturated, linear and/or branched alkanecarboxylic acids having 3 to 30 C atoms with saturated and/or unsaturated, linear and/or branched alcohols having 3 to 30 C atoms, and esters of aromatic carboxylic acids with saturated and/or unsaturated, linear and/or branched alcohols having 3 to 30 C atoms, selected especially from the group comprising isopropyl myristate, isopropyl stearate, isopropyl palmitate, isopropyl oleate, n-butyl stearate, n-hexyl laurate, n-decyl laurate, isooctyl stearate, isononyl stearate, isononyl isononanoate, 2-ethylhexyl palmitate, 2-ethylhexyl laurate, 2-ethylhexyl ethylhexanoate, cetearyl 2-ethylhexanoate, 3,5,5-trimethylhexyl 3,5,5-trimethylhexanoate, 2-ethylhexyl isononanoate, 2-ethylhexyl 3,5,5-trimethylhexanoate, 2-ethylhexyl 2-ethylhexanoate, 2-hexyldecyl stearate, 2-octyldecyl palmitate, oleyl oleate, oleyl erucate, erucyl oleate, erucyl erucate and synthetic or natural mixtures of such esters), fats, waxes and other natural and synthetic fatty substances, preferably esters of fatty acids with alcohols of low C number (e.g. with isopropanol, propylene glycol or glycerol) or esters of fatty alcohols with alkanoic acids of low C number or with fatty acids, alkyl benzoates (e.g. mixtures of n-dodecyl, n-tridecyl, n-tetradecyl and n-pentadecyl benzoate) and cyclic or linear silicone oils (e.g. dimethylpolysiloxanes, diethyl polysiloxanes, diphenylpolysiloxanes and mixed forms thereof).

Nurturing substances which are outstandingly suitable as or for combination with the formulations used according to the invention also include the following:
- waxes, e.g. candelilla wax or carnauba wax,
- ceramides, these being understood as meaning N-acylsphingosines (fatty acid amides of sphingosine) or synthetic analogues of such lipids (so-called pseudoceramides), which markedly improve the water retention capacity of the stratum corneum,
- phospholipids, for example soya lecithin, egg lecithin and kephalins,
- petrolatum and paraffin and silicone oils, the latter including, inter alia, dialkylsiloxanes and alkylarylsiloxanes, such as dimethylpolysiloxane and methylphenylpolysiloxane, and their alkoxylated and quaternized derivatives.

An aqueous phase of a formulation to be used in the pig skin model according to the invention can advantageously be or comprise alcohols, diols or polyols of low C number, and their ethers, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, and alcohols of low C number, e.g. ethanol, isopropanol,1,2-propanediol and glycerol, and especially one or more thickeners which can advantageously be selected from the group comprising silicon dioxide, aluminium silicates, polysaccharides or derivatives thereof, e.g. hyaluronic acid, xanthan gum and hydroxypropyl methyl cellulose, and particularly advantageously from the group comprising polyacrylates, preferably a polyacrylate from the group comprising so-called carbopols, e.g. carbopols of types 980, 981, 1382, 2984 and 5984, each individually or in combination.

Mixtures to be used according to the invention that are in the form of an emulsion advantageously comprise one or more emulsifiers. O/W emulsifiers can advantageously be selected e.g. from the group comprising polyethoxylated, polypropoxylated or polyethoxylated and polypropoxylated products, for example:
- fatty alcohol ethoxylates,
- ethoxylated wool wax alcohols,
- polyethylene glycol ethers of the general formula R-O-(-CH₂-CH₂-O-)ₙ-R',
- fatty acid ethoxylates of the general formula R-COO-(-CH₂-CH₂-O-)ₙ-H,
- etherified fatty acid ethoxylates of the general formula R-COO-(-CH₂-CH₂-O-)ₙ-R',
- esterified fatty acid ethoxylates of the general formula R-COO-(-CH₂-CH₂-O-)ₙ-C(O)-R',
- polyethylene glycol glycerol fatty acid esters,
- ethoxylated sorbitan esters,
- cholesterol ethoxylates,
- ethoxylated triglycerides,
- alkyl ether carboxylic acids of the general formula R-COO-(-CH₂-CH₂-O-)ₙ-OOH, n being a number from 5 to 30,
- polyethoxylated sorbitol fatty acid esters,
- alkyl ether sulfates of the general formula R-O-(-CH₂-CH₂-O-)ₙ-SO₃-H,
- fatty alcohol propoxylates of the general formula R-O-(-CH₂-CH(CH₃)-O-)ₙ-H,
- polypropylene glycol ethers of the general formula R-O-(-CH₂-CH(CH₃)-O-)ₙ-R',
- propoxylated wool wax alcohols,
- etherified fatty acid propoxylates R-COO-(-CH₂-CH(CH₃)-O-)ₙ-R',
- esterified fatty acid propoxylates of general formula R-COO-(-CH₂-CH(CH₃)-O-)ₙ-C(O)-R',
- fatty acid propoxylates of the general formula R-COO-(-CH₂-CH(CH₃)-O-)ₙ-H,
- polypropylene glycol glycerol fatty acid esters,
- propoxylated sorbitan esters,
- cholesterol propoxylates,
- propoxylated triglycerides,
- alkyl ether carboxylic acids of the general formula R-O-(-CH₂-CH(CH₃)-O-)ₙCH₂-COOH,
- alkyl ether sulfates or the corresponding acids of the general formula R-O-(-CH₂-CH(CH₃)-O-)ₙ-SO₃-H,
- fatty alcohol ethoxylates/propoxylates of the general formula R-O-Xₙ-Yₘ-H,
- polypropylene glycol ethers of the general formula R-O-xₙ-Yₘ-R',
- etherified fatty acid propoxylates of the general formula R-COO-Xₙ-Yₘ-R',
- fatty acid ethoxylates/propoxylates of the general formula R-COO-Xₙ-Yₘ-H.

According to the invention, the polyethoxylated, polypropoxylated or polyethoxylated and polypropoxylated O/W emulsifiers used are particularly advantageously selected from the group of substances with HLB values of 11 - 18, and very particularly advantageously from those with HLB values of 14.5 - 15.5, if they contain saturated radicals R and R'. If the O/W emulsifiers contain unsaturated radicals R and/or R', or if isoalkyl derivatives are present, the preferred HLB value of such emulsifiers can also be lower or higher.

The fatty alcohol ethoxylates are advantageously selected from the group comprising ethoxylated stearyl alcohols, cetyl alcohols and cetylstearyl alcohols (cetearyl alcohols). The following are particularly preferred: polyethylene glycol (13) stearyl ether (steareth-13), polyethylene glycol (14) stearyl - ether (steareth-14), polyethylene glycol (15) stearyl ether (steareth-15), polyethylene glycol (16) stearyl ether (steareth-16), polyethylene glycol (17) stearyl ether (steareth-17), polyethylene glycol (18) stearyl ether (steareth-18), polyethylene glycol (19) stearyl ether (steareth-19), polyethylene glycol (20) stearyl ether (steareth-20), polyethylene glycol (12) isostearyl ether (isosteareth-12), polyethylene glycol (13) isostearyl ether (isosteareth-13), polyethylene glycol (14) isostearyl ether (isosteareth-14), polyethylene glycol (15) isostearyl ether (isosteareth-15), polyethylene glycol (16) isostearyl ether (isosteareth-16), polyethylene glycol (17) isostearyl ether (isosteareth-17), polyethylene glycol (18) isostearyl ether (isosteareth-18), polyethylene glycol (19) isostearyl ether (isosteareth-19), polyethylene glycol (20) isostearyl ether (isosteareth-20), polyethylene glycol (13) cetyl ether (ceteth-13), polyethylene glycol (14) cetyl ether (ceteth-14), polyethylene glycol (15) cetyl ether (ceteth-15), polyethylene glycol (16) cetyl ether (ceteth-16), polyethylene glycol (17) cetyl ether (ceteth-17), polyethylene glycol (18) cetyl ether (ceteth-18), polyethylene glycol (19) cetyl ether (ceteth-19), polyethylene glycol (20) cetyl ether (ceteth-20), polyethylene glycol (13) isocetyl ether (isoceteth-13), polyethylene glycol (14) isocetyl ether (isoceteth-14), polyethylene glycol (15) isocetyl ether (isoceteth-15), polyethylene glycol (16) isocetyl ether (isoceteth-16), polyethylene glycol (17) isocetyl ether (isoceteth-17), polyethylene glycol (18) isocetyl ether (isoceteth-18), polyethylene glycol (19) isocetyl ether (isoceteth-19), polyethylene glycol (20) isocetyl ether (isoceteth-20), polyethylene glycol (12) oleyl ether (oleth-12), polyethylene glycol (13) oleyl ether (oleth-13), polyethylene glycol (14) oleyl ether (oleth-14), polyethylene glycol (15) oleyl ether (oleth-15), polyethylene glycol (12) lauryl ether (laureth-12), polyethylene glycol (12) isolauryl ether (isolaureth-12), polyethylene glycol (13) cetylstearyl ether (ceteareth-13), polyethylene glycol (14) cetylstearyl ether (ceteareth-14), polyethylene glycol (15) cetylstearyl ether (ceteareth-15), polyethylene glycol (16) cetylstearyl ether (ceteareth-16), polyethylene glycol (17) cetylstearyl ether (ceteareth-17), polyethylene glycol (18) cetylstearyl ether (ceteareth-18), polyethylene glycol (19) cetylstearyl ether (ceteareth-19), polyethylene glycol (20) cetylstearyl ether (ceteareth-20).

The fatty acid ethoxylates can also advantageously be selected from the following group: polyethylene glycol (20) stearate, polyethylene glycol (21) stearate, polyethylene glycol (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate, polyethylene glycol (15) oleate, polyethylene glycol (16) oleate, polyethylene glycol (17) oleate, polyethylene glycol (18) oleate, polyethylene glycol (19) oleate, polyethylene glycol (20) oleate.

Sodium laureth-11 carboxylate can advantageously be used as an ethoxylated alkyl ether carboxylic acid or a salt thereof. Sodium laureth-1-4 sulfate can advantageously be used as an alkyl ether sulfate. Polyethylene glycol (30) cholesteryl ether can advantageously be used as an ethoxylated cholesterol derivative. Polyethylene glycol (25) soya sterol has also proved valuable.

Polyethylene glycol (60) evening primrose glycerides can advantageously be used as ethoxylated triglycerides.

It is also advantageous to select the polyethylene glycol glycerol fatty acid esters from the group comprising polyethylene glycol (20) glyceryllaurate, polyethylene glycol (21) glyceryllaurate, polyethylene glycol (22) glyceryllaurate, polyethylene glycol (23) glyceryllaurate, polyethylene glycol (6) glycerylcaprylate/caprate, polyethylene glycol (20) glyceryloleate, polyethylene glycol (20) glycerylisostearate and polyethylene glycol (18) glyceryloleate/cocoate.

It is likewise favourable to select the sorbitan esters from the group comprising polyethylene glycol (20) sorbitan monolaurate, polyethylene glycol (20) sorbitan monostearate, polyethylene glycol (20) sorbitan mono isostearate, polyethylene glycol (20) sorbitan monopalmitate and polyethylene glycol (20) sorbitan monooleate.

The following can be used as advantageous W/O emulsifiers: fatty alcohols having 8 to 30 carbon atoms, monoglyceryl esters of saturated and/or unsaturated, branched and/or unbranched alkanecarboxylic acids with a chain length of 8 to 24 C atoms, especially 12 to 18 C atoms, diglyceryl esters of saturated and/or unsaturated, branched and/or unbranched alkanecarboxylic acids with a chain length of 8 to 24 C atoms, especially 12 to 18 C atoms, monoglyceryl ethers of saturated and/or unsaturated, branched and/or unbranched alcohols with a chain length of 8 to 24 C atoms, especially 12 to 18 C atoms, diglyceryl ethers of saturated and/or unsaturated, branched and/or unbranched alcohols with a chain length of 8 to 24 C atoms, especially 12 to 18 C atoms, propylene glycol esters of saturated and/or unsaturated, branched and/or unbranched alkanecarboxylic acids with a chain length of 8 to 24 C atoms, especially 12 to 18 C atoms, and sorbitan esters of saturated and/or unsaturated, branched and/or unbranched alkanecarboxylic acids with a chain length of 8 to 24 C atoms, especially 12 to 18 C atoms.

Particularly advantageous W/O emulsifiers are glyceryl monostearate, glyceryl monoisostearate, glyceryl monomyristate, glyceryl monooleate, diglyceryl - monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprate and glyceryl monocaprylate.

Mixtures to be used according to the invention (e.g. a topical cosmetic formulation) advantageously contain cooling agents. The following may be mentioned as examples of cooling agents: I-menthol, d-menthol, racemic menthol, menthone glyceryl acetal, menthyl lactate, substituted menthyl-3-carboxamides (e.g. menthyl-3-carboxylic acid N-ethylamide), 2-isopropyl-N-2,3-trimethylbutanamide, substituted cyclohexanecarboxamides, 3-menthoxypropane-1,2-diol, 2-hydroxyethyl menthyl carbonate, 2-hydroxypropyl menthyl carbonate, N-acetylglycine menthyl ester, isopulegol, hydroxycarboxylic acid menthyl esters (e.g. menthyl 3-hydroxybutyrate), monomenthyl succinate, 2-mercaptocyclodecanone, menthyl 2-pyrrolidin-5-one-carboxylate, 2,3-dihydroxy-p-menthane, 3,3,5-trimethylcyclohexanone glyceryl ketal, 3-menthyl-3,6-dioxaalkanoates and -trioxaalkanoates, 3-menthyl methoxyacetate and icilin.

### Antimicrobials and preservatives

The formulations used according to the invention (e.g. topical cosmetic formulations) also advantageously contain antimicrobial substances. Other active substances worthy of particular mention in addition to conventional preservatives, i.e. in addition to the large group of conventional antibiotics, are the products relevant to cosmetics, such as triclosan, climbazole, zinc pyrithione, ichthyol, octopirox (2-aminoethanol salt of 1-hydroxy-4-methyl-6-(2,4,4-trimethylpentyl)-2(1 H)-pyridone), chitosan, farnesol, octyloxyglycerol, glyceryl monolaurate, arylalkyl alcohols, e.g. phenylethyl alcohol, 3-phenyl-1-propanol, veticol or muguet alcohol, and aliphatic diols, e.g. 1,2-decanediol, or combinations of said substances which are used, inter alia, to combat armpit odour, foot odour or scaling.

Aryl-substituted or aryloxy-substituted, unbranched or monoalkyl- or polyalkyl-branched, saturated or unsaturated fatty alcohols, aldehydes, acids and acid esters, alkanediols, dialdehydes, dicarboxylic acids and dicarboxylic acid esterswith chain lengths of C₂ to C₄₀ from a synthetic or natural source (e.g. from coconut fat, palm kernel fat, wool wax, lanolin).

Monohydroxy and oligohydroxy fatty acids with chain lengths of C₂ to C₂₄ (e.g. lactic acid, 2-hydroxypalmitic acid), their oligomers and/or polymers and vegetable and animal raw materials containing these. Ethoxylated, propoxylated or mixed ethoxylated/ propoxylated cosmetic fatty alcohols, fatty acids and fatty acid esters with chain lengths of C₂ to C₄₀ and having 1 to 150 EO and/or PO units. It is also possible to use so-called "natural" antibacterial substances, most of which are ethereal oils. Examples of typical antibacterially active oils are those of anise, lemon, orange, rosemary, wintergreen, clove, thyme, lavender, hops, citronella, wheat, lemongrass, cedarwood, cinnamon, geranium, sandalwood, violet, eucalyptus, peppermint, gum benzoin, basil and fennel, as well as Ocmea origanum, Hydastis carradensis, Berberidaceae daceae, Ratanhiae or Curcuma longa.

Examples of important antimicrobially active substances which can be found in ethereal oils are anethole, catechol, camphene, carvacrol, eugenol, eucalyptol, ferulic acid, farnesol, hinokitiol, tropolone, limonene, menthol, methyl salicylate, thymol, terpineol, verbenone, berberin, curcumin, caryophyllene oxide, nerolidol and geraniol.

It is also possible to use mixtures of said active systems or active substances, as well as active substance combinations containing these active substances.

The amount of active substances in the preparations is preferably 0.01 to 20 weight percent and particularly preferably 0.05 -10 weight percent, based on the total weight of the preparations. Furthermore, a mixture to be used according to the invention can also be combined with sweat-inhibiting substances (antiperspirants) and odour absorbers. The antiperspirants used are primarily aluminium salts such as aluminium chloride, aluminium chlorohydrate, nitrate, sulfate, acetate, etc., and also aluminium hydroxy-chlorides. In addition to these, however, it can also be advantageous to use zinc, magnesium and zirconium compounds. The following can also be used: a) protein-precipitating substances such as, inter alia, formaldehyde, glutaraldehyde, natural and synthetic tannins and trichloroacetic acid, which bring about a surface occlusion of the sweat glands, b) local anaesthetics (inter alia, dilute solutions of e.g. lidocaine, prilocaine or mixtures of such substances), which switch off the sympathetic supply to the sweat glands by blocking the peripheral nerve paths, c) zeolites of the X, A or Y type, which, in addition to reducing sweat secretion, also act as adsorbents of bad odours, and d) botulinum toxin (toxin of the bacterium *Chlostridium botulinum*), and other substances that block the release of the transmitter substance acetylcholine relevant to sweat secretion.

Examples of odour absorbers are the sheet silicates described in Offenlegungsschrift DE-P 40 09 347, especially montmorillonite, kaolinite, nontronite, saponite, hectorite, bentonite and smectite, and also e.g. zinc salts of ricinoleic acid. They also include deodorants, bactericidal or bacteriostatic deodorizing substances, e.g. hexachlorophene, 2,4,4'-trichloro-2'-hydroxydiphenyl ether (Irgasan), 1,6-di(4-chlorophenylbiguanido)hexane (chlorhexidine), 3,4,4'-trichlorocarbanilide, and the active agents described in Offenlegungsschriften DE-37 40 186, DE-39 38 140, DE-42 04 321, DE-42 29 707, DE-42 29 737, DE-42 37 081, DE-43 09 372 and DE-43 24 219, which contain cationic substances, e.g. quaternary ammonium salts, and odour absorbers, e.g. ^{(R)}Grillocin (combination of zinc ricinoleate and various additives) or triethyl citrate, optionally in combination with ion exchange resins.

The amount of deodorizing and/or antiperspirant substances in the mixtures is preferably 0.01 to 20 weight percent and particularly preferably 0.05 - 10 weight percent, based on the total weight of the preparations.

In numerous cases, the mixtures to be used in the formulations, according to the invention, can also advantageously be combined with preservatives. It is preferable here to choose preservatives like benzoic acid and its esters and salts, propionic acid and its salts, salicylic acid and its salts, 2,4-hexadienoic acid (sorbic acid) and its salts, formaldehyde and paraformaldehyde, 2-hydroxydiphenyl ether and its salts, zinc 2-sulfidopyridine N-oxide, inorganic sulfites and bisulfites, sodium iodate, chlorobutanolum, 4-ethylmercury(II)-5-amino-1,3-bis(2-hydroxybenzoic acid) and its salts and esters, dehydroacetic acid, formic acid, 1,6-bis(4-amidino-2-bromophenoxy)-n-hexane and its salts, the sodium salt of ethylmercury(II)-thiosalicylic acid, phenylmercury and its salts, 10-undecylenic acid and its salts, 5-amino-1,3-bis(2-ethylhexyl)-5-methylhexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)urea, 4-chloro-m-cresol, 2,4,4'-trichloro-2'-hydroxydiphenyl ether, 4-chloro-3,5-dimethylphenol, 1,1'-methylenebis(3-(1-hydroxymethyl-2,4-dioximidazolidin-5-yl)urea), poly(hexamethylenediguanide) hydrochloride, 2-phenoxyethanol, hexamethylenetetramine, 1-(3-chloroallyl)-3,5,7-triaza-1-azoniaadamantane chloride, 1-(4-chlorophenoxy)-1-(1H-imidazol-1-yl)-3,3-dimethyl-2-butanone, 1,3-bis(hydroxymethyl)-5,5-dimethyl-2,4-imidazoli-dinedione, benzyl alcohol, octopirox, 1,2-dibromo-2,4-dicyanobutane, 2,2'-methylenebis(6-bromo-4-chlorophenol), bromochlorophene, mixture of 5-chloro-2-methyl-3(2H)-isothiazolinone and 2-methyl-3(2H)-isothiazolinone with magnesium chloride and magnesium nitrate, 2-benzyl-4-chlorophenol, 2-chloroacetamide, chlorhexidine, chlorhexidine acetate, chlorhexidine gluconate, chlorhexidine hydrochloride, 1-phenoxypropan-2-ol, N-alkyl(C₁₂-C₂₂)-trimethylammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxymethylurea, 1,6-bis(4-amidinophenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, **alkyl(C₈-**C₁₈)dimethylbenzylammonium chloride, alkyl(C₈-C₁₈)dimethylbenzylammonium bromide, alkyl(C₈-C₁₈)dimethylbenzylammonium saccharinate, benzyl hemiformal, 3-iodo-2-propynylbutyl carbamate or sodium hydroxymethylaminoacetate.

Formulations used in the pig skin model according to the invention, especially dermatological formulations, can also advantageously contain dyestuffs and/or coloured pigments, especially if they are to be used in the decorative cosmetics sector. The dyestuffs and coloured pigments can be selected from the appropriate positive list of the cosmetics regulations or from the EC list of cosmetic colourants. In most cases they are identical to the dyestuffs permitted for foods. Examples of advantageous coloured pigments are titanium dioxide, mica, iron oxides (e.g. Fe₂O₃, Fe₃O₄, FeO(OH)) and/or tin oxide. Examples of advantageous dyestuffs are carmine, Berlin blue, chromium oxide green, ultramarine blue and/or manganese violet. Mixtures of said active systems can also be used.

The invention is further described by the figures and following examples, none of which shall be construed as limiting the scope of claims:

### Examples

### Example 1:

### Skin cell proliferation

Skin cell viability of Pietrain and Landrace domestic pigs was measured on dorsal skin samples of 4 x 4 x 3 mm (length x width x thickness). The samples were obtained from freshly slaughtered animals sacrificed with a captive bolt pistol coupled with exsanguination (Italian law R.D. 20.12.1928 n.3298). The area of the skin to be excised was washed with distilled water, and then dried with sterile gauzes. Afterwards, the hair shafts were cut by using an electric clipper at a length of 1 mm. Subsequently, all the dorsal skin was washed by using a surgical soap that was then removed by plugging with sterile gauzes soaked in distilled water. Afterwards, the skin was further cleaned with chlorhexidine and then dried with sterile gauzes. Lastly, the skin was restored by using a physiological solution.

Patches of skin were excised with a scalpel in order to obtain lens-shaped patches having the dimension of approximately 6 cm (length) and 4 cm (width). The excision was executed by performing two cuts that follow the edges of the skin lens-shaped patch to be prepared.Then, the patch was lifted carefully by the use of tweezers and, by using a scalpel, the skin including epidermis, dermis and fat layer was gently detached from the muscle. The fat layer was further reduced to a maximum thickness of 3 mm to which allows optimal nutrition of the tissue from the culture medium.

Once excised, the skin patch was placed in a 50 ml tube containing 40 ml of transport medium and maintained at 4 °C until the sample arrived at the laboratory to be further processed. The transport medium was prepared by adding 10 percent of penicillin/streptomycin to DMEM. This medium guarantees skin tissue characteristics and protects the samples from bacterial contamination during the transfer from the slaughterhouse to the laboratory. Upon arrival at the laboratory, the skin patches were transferred into William's E medium as the culture medium. Skin viability was controlled by verifying the rate of both skin and hair cell apoptosis and proliferation. The skin is strictly kept on sterile environment. This procedure assures acceptable conditions of skin viability up to 21 days.

The skin samples to be used for the various experimental proceeding were processed as follows: The edges of skin patches were cut and discarded and the remaining skin sample was placed in a base of sterilized cork and then cut to a size of 4 x 4 x 3 mm (length x width x thickness).

The skin samples were cultured by setting-up an air-liquid interface system. A six well plate was prepared by placing at the bottom of each well a sterilized cotton pad and 5 ml of culture medium. With the epidermis surface upward and fat/dermis downward, two samples per well were placed on the cotton pad soaked with culture medium. The culture medium was replaced every 3 days. The results are shown in Table 2:

**Table 2**

| Skin cell proliferation | | | | |
|---|---|---|---|---|
| | Day 0 | | Day 6 | |
| | Sample A: 30 ±5 follicles/cm² | Sample B: 11 ±3 follicles/cm² | Sample A: 30 ±5 follicles/cm² | Sample B: 11 ±3 follicles/cm² |
| % of Ki-67 positive cells | 5.37 ±0.76 | 2.74 ± 0.37 | 4.03 ± 0.20 | 0.98 ± 0.05 |
| % Proliferation sample A versus sample B day 0 | 100 | 51 | 75 | 18 |

### Example 2

### Skin sample viability of skin samples according to the invention

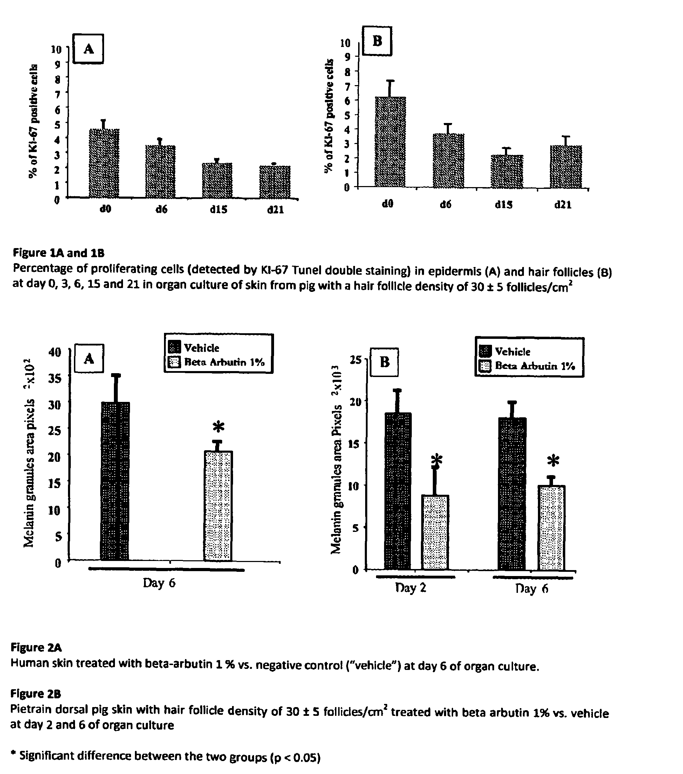

Skin samples of Pietrain and Landrace domestic pigs were obtained and treated as given in Example 1. Skin cell proliferation was measured by detecting Ki-67 positive cells in the manner described in Example 1. **Figure 1A** shows that the percentage of proliferating hair matrix cells in the skin samples' epidermis had only roughly halved and was significantly higher than 1.5 % Ki-67 positive cells even at day 21 of cultivation, indicating that the epidermis skin cells were still viable after 21 days. Likewise, **Figure 1B** shows that the percentage of proliferating cells in the skin sample's hair follicles had only roughly halved and was significantly higher than 1.5 % even at day 21 of cultivation, indicating that the hair follicles' skin cells were still viable after 21 days.

### Example 3

### Use of pig skin for the screening of potential modulator of skin pigmentation

For this experiment, full thickness pig skin has been excised from dorsal part of a pig with 30 ±5 follicles/cm² (age: 8 weeks; weight: 24 kg). The skin samples have been cut under sterile condition in smaller pieces (surface: 4 x 4; thickness: 3 mm) and placed in a 6 wells plate (two samples per well) over a sterilized cotton immerse in 5 ml of culture medium, incubated at 37 °C, 5 % CO₂, 100 % humidity, as described above. Culture medium has been renewed every other day. Beta arbutin (hydroquinone glucoside), a recognized inhibitor of tyrosinase, has been selected as positive control for skin lightening, In fact, in full thickness human skin, beta arbutin has shown to significantly inhibit skin pigmentation by 31 % (Figure 2a). Thus, 10 µl of beta arbutin (1 % w/v in culture medium) per sample has been added topically and re-applied daily. As negative control, culture medium has been applied topically by following the same experimental procedures as for beta-arbutin. Six pig skin samples have been used for each treatment (i.e. beta arbutin and culture medium). The pig skin samples were incubated for either 2 or 6 days respectively.

Following organ culture procedure, skin samples have been embedded in cryomedium by using liquid nitrogen. Embedded samples have been cut in thin slides (thickness: 7 µm) and were then hisotochemically stained by Fontana-Masson technique, that stains argentaffin melanin granules in black and the cells' nuclei in pink.

The amount of melanin present in each slide has been evaluated by measuring the areas covered by the black spots, by using the software ImageJ (National Institute of Health, US). The area is proportional to the amount of melanin present in the tissue. The data from all samples of the same experimental group have been pooled and the means plus or minus SEM calculated. The statistical significance (P-value) was been determined by Mann-Whitney U Test for nonparametric samples. The difference between two groups had been considered significant when p less than 0.05. After 2 and 6 days of application of 1 % beta-arbutin, the decrease of pigmentation has been 52.4 % and 44.2 % respectively, in comparison to the negative control (**Figure 2b**). Thus, as for human skin, beta-arbutin strongly inhibits pig skin sample pigmentation, thus indicating the reliability of this model.

### Example 4

### Use of baby pig skin for the screening of potential modulator of hair growth

For this experiment, full thickness skin samples had been excised from dorsal part of a pig with 30 plus or minus 5 follicles/cm² (age: 8 weeks; weight: 22 kg). The pig skin has been prepared and cultured as described in the previous example. Since the increase in the proliferation of hair follicle matrix cells as well as the decrease of apoptosis is one of the earliest event that can be observed in case of modulation of hair growth, this parameter can be used to assess the potential efficacy of a candidate modulator of hair growth. As a consequence, changes in the proliferation rate of hair follicles matrix cells have been investigated in pig skin organ culture following incubation with recognized modulators of hair growth. Cyclosporin A (CsA) and transforming growth factor beta 2 (TGFb2), have been selected as standard stimulator and inhibitor of hair growth, respectively. As a final concentration either 100ng/ml CsA or 25ng/ml TGFb2 has been added to the culture medium, in order to simulate and inhibit, respectively the systemic application. Two different experimental procedures have been investigated:
(i) In the first experiment, the pig skin samples had been incubated with the potential modulators from day 0 to day 6.
(ii) In the second experiment, the skin had been kept in culture until day 14. Then, the skin has been incubated with the potential modulators up day 21. The aim of this protocol has been to verify if the skin would be still reactive following long time culture.

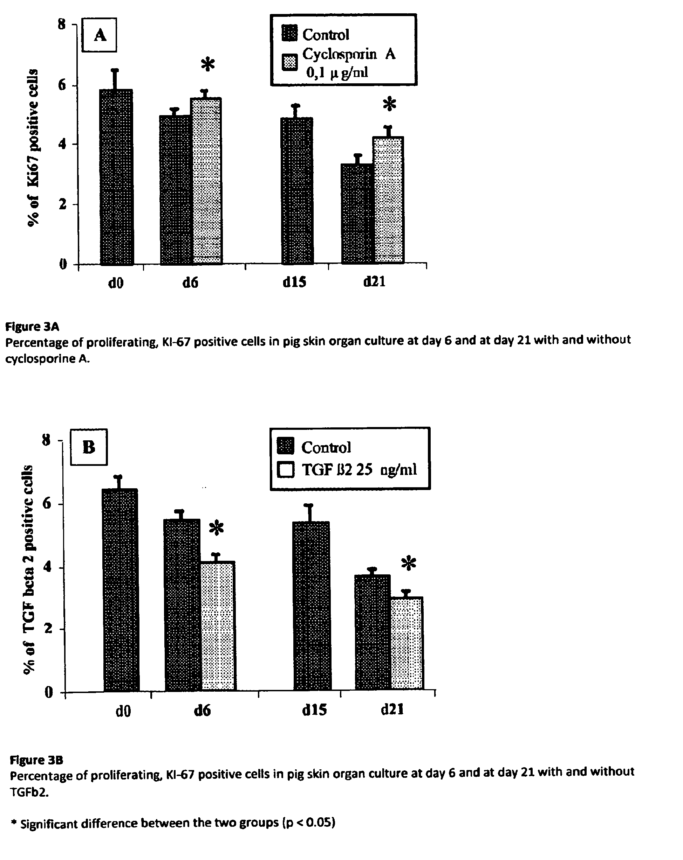

Following the organ culture procedure, skin samples had been embedded in cryomedium by using liquid nitrogen. Embedded samples had been cut in thin slides (thickness: 7 µm) and were then immunolabelled by performing Ki67/DAPI double staining to detect proliferative cells localized on the bulb region. In particular, while Ki67 labels proliferating cells, DAPI stains all cell nuclei, Quantitative analysis has been performed by using a custom modified version of the software ImageJ (National Institute of Health, US). The data from all samples of the same experimental group have been pooled and the means plus or minus SEM calculated. The statistical significance (P-value) has been determined by Mann-Whitney U test for nonparametric samples. The difference between two groups has been considered significant when p less than 0.05. As shown in **Figure 3****,** in both experiments, CsA and TGFb2 have shown to stimulate and inhibit, respectively, hair matrix cell proliferation, suggesting to have exerted the expected modulatory action on hair growth. In particular, CsA has shown to increase cell proliferation at day 6 and day 21 of 10.1 % and 19.8 % respectively, while TGFb2 has inhibited it of 24.8 % and 36.1 % respectively. The effects of both agents were statistically significant versus the untreated control.

### Example 5

### Influence of fat layer on skin sample viability

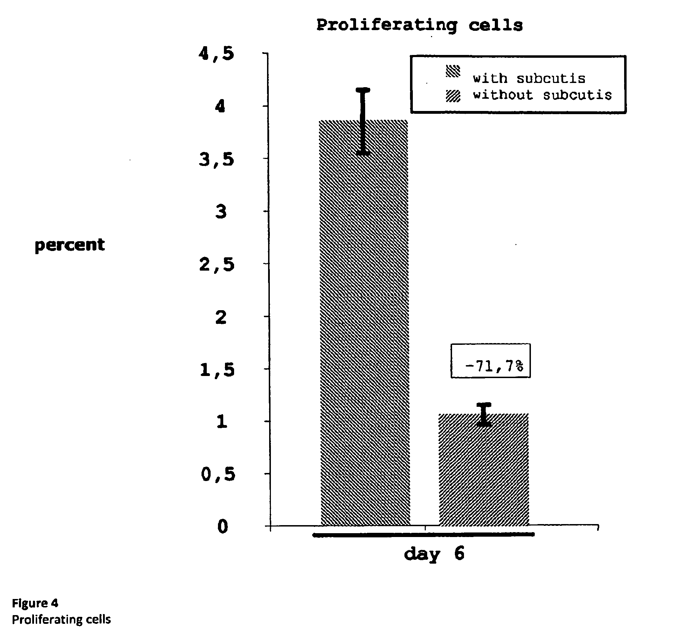

Two sets of pig skin samples a were obtained as given in Example 1. Pig skin samples of the first group did comprise a subcutis layer and had a total thickness of 3,5 mm. Pig skin sample of the second group (control) had their subcutis layer substantially removed, sample thickness was 2 mm. Both skin sample groups were subjected to the same cultivation conditions: In a 6 well plate, two skin samples per well were placed on a cotton pad with the epidermis layer facing upward. Each well was filled with 5 ml of William's E culture medium, such that the surface of the epidermis layer was in contact with surrounding air, 6 samples for each group (test and control group) were tested. The medium was changed every other day. The 6 well plate was kept in an incubator at 37 °C, 100 % humidity and 5 % CO₂. After 6 days, the samples were embedded in Cryomatrix medium (Shandon, USA) and cut into 7 micrometres thick slides, stained with Ki67-DAPI double immunolabelling agent and evaluated by comparing the proliferating cell counts (cf. **Figure 4**). It shows that pig skin samples with subcutis had a significant higher percentage of Ki-67 positive cells compared to the control samples without subcutis.

### Example 6

### Structure of an average pig skin sample according to the present invention

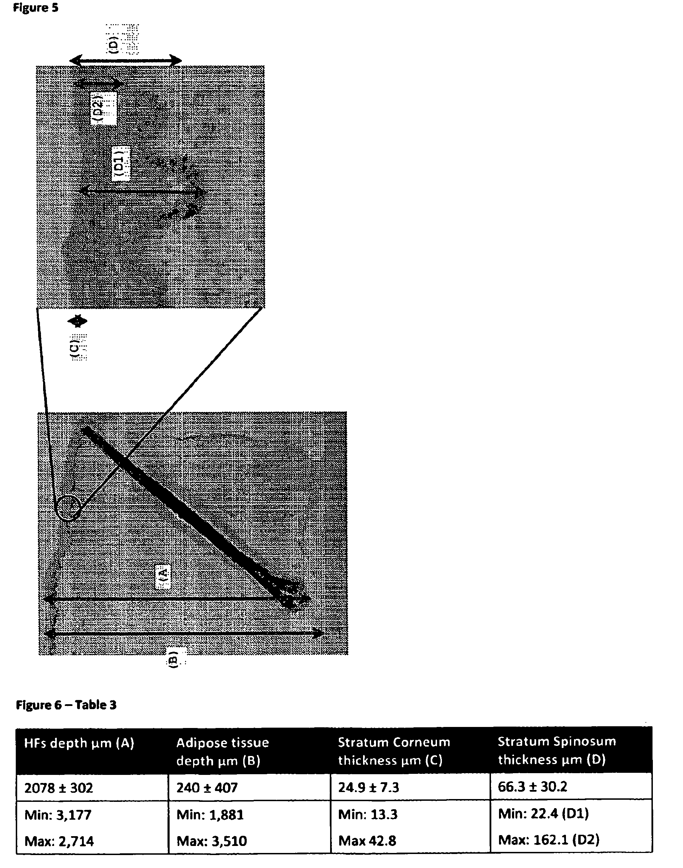

As depicted in **Figures 5 and 6****,** a pig skin sample of the present invention comprises an epidermis layer with a stratum corneum and stratum spinosum layer, a dermis layer and a sub-cutis layer with a hair bulb and surrounding hair follicle. The stratum corneum has an average thickness of 24,9 µm ± 7,3 µm; a minimum thickness of 13,3 µm and a maximum thickness of 42,8 µm is frequently observed. Likewise, the stratum spinosum thickness is on average 68,3 ± 30,2 µm, with a minimum thickness for valleys of the epidermis (dimension D1 in **Figure 5**) of 22,4 µm and maximum thickness at "hill regions" of the dermis (dimension D2 of Figure 5) of 162,1 µm being frequently encountered. Hair follicles typically have a distance to the epidermis surface of 1,38 µm to 2,71 µm, with an average distance of 2,1 mm ± 302 µm. Thus, depending on the hair follicle distance to the epidermis surface, the total thickness of the skin sample can be 1,88 µm (for hair follicles close to the epidermis surface), but frequently is 2,4 mm ± 407 µm and can even be 3,5 mm. The aforementioned dimensions are an average for Pietrain and Landrace pig skin. However, the dimensions can vary for other pig skin races.

## Claims

1. Use of a pig skin sample taken from a pigs' neck and/or dorsal part having a hair follicle density of at least 15 follicles per cm² for simulating or analyzing a treatment of human or animal skin, wherein the pig skin sample comprises an epidermis, dermis and subcutis layer.

2. Use according to Claim 1, wherein the subcutis layer comprises a hair follicle.

3. Method for modelling, simulating or analyzing a selected effect of a selected treatment of human or animal skin, comprising the steps of:
(a) Providing a skin sample taken from a pigs' neck and/or dorsal part having a hair follicle density of at least 15 follicles per cm² of a pig, the sample including an epidermis, dermis and a subcutis layer;
(b) Subjecting the sample to the selected treatment; and
(c) Observing the effect of the treatment on the skin sample.

4. Method of Claim 3, wherein the skin sample comprises at least one primary hair follicle.

5. Method of any of Claims 3 to 4, wherein the skin sample is a skin sample of a domestic pig.

6. Method of Claim 5, wherein the skin sample is a skin sample of a Pietrain or Landrace pig.

7. Method of any of Claims 3 to 6, wherein the skin sample comprises a subcutis layer with an average thickness of at least 1 mm.

8. Method of any of Claims 3 to 7, wherein the skin sample has a dimension (length x width x thickness) of (3-6) x (3-6) x (2-5) mm.

9. Method of any of Claims 3 to 8, wherein the effects to be observed are
(i) effects of exposition of the skin sample to substances, the substances being applied topically to the skin sample and/or systemically to the skin sample by mixing with a culture medium, and/or
(ii) effects of skin sample irradiation.

10. Method of any of Claims 3 to 9 for the assessment of:
• modulation of skin and/or hair pigmentation
• modulation of growth;
• modulation of skin and/or hair viability and/or proliferation;
• modulation of fat metabolism;
• anti-cellulitis properties of substances;
• slimming;
• anti-ageing effects, particularly by fat cell stimulation;
• allergenic potential and/or irradiation;
• UV-protection, particularly UV erythema prevention, alleviation and healing;
• modulation of connecting tissue properties, particularly for the assessment of anti-wrinkle properties of substances;
• anti-oxidative effects;
• wound healing;
• modulation of skin barrier function;
• modulation of ion channels, especially neurofunctional channels and preferably channels activated by GABA, glutamate, acetylcholine, serotonin, adrenalin and ATP, and temperature sensitive channels (TRPM8, TRPV3, TRPV4, TRPV1, ANKTM, TRVP2);
• immunestimulation; immunesuppression;
• sebum stimulation, sebum suppression;
• anti-microbial effectiveness, particularly anti-acne effectiveness;
• sweat secretion decrease;
• substantivity of materials on a skin surface;
• film forming effectiveness;
• modulation of hair thickness;
• moisturization;
• phototoxicity;
• skin metabolism of compounds.

11. System for modeling a selected effect of a selected treatment of human skin, comprising an isolated skin sample taken from a pig's neck and/or dorsal part having a hair follicle density of at least 15 follicles per cm² of a pig placed with its subcutis layer on a support comprising a cultivation medium, the sample including an epidermis, dermis and subcutis layer.

12. System according to Claims 11, wherein the skin sample is a skin sample of a domestic pig.

## Patentansprüche

1. Verwendung einer Schweinehautprobe entnommen vom Nacken oder dem Bauch eines Schweines mit einer Haarfollikeldichte von mindestens 15 Follikeln pro cm² zur Simulation oder Analyse bei der Behandlung von menschlicher oder tierischer Haut, wobei die Schweinehautprobe eine Epidermis, eine Dermis und eine Subkutanschicht enthält.

2. Verwendung nach Anspruch 1, wobei die Subkutanschicht Haarfollikel enthält.

3. Verfahren zur Modellierung, Simulation oder Analyse eines ausgewählten Effektes bei der ausgewählten Behandlung von menschlicher oder tierischer Haut umfassend die folgenden Schritte:
(a) Bereitstellen einer Schweinehautprobe entnommen vom Nacken oder dem Bauch eines Schweines mit einer Haarfollikeldichte von mindestens 15 Follikeln pro cm², wobei die Schweinehautprobe eine Epidermis, eine Dermis und eine Subkutanschicht enthält.
(b) Unterwerfung der Probe der ausgewählten Behandlung, und
(c) Verfolgung des Effektes, den die Behandlung auf die Hautprobe ausübt.

4. Verfahren nach Anspruch 3, wobei die Hautprobe mindestens ein primäres Haarfollikel enthält.

5. Verfahren nach einem der Ansprüche 3 und 4, wobei die Hautprobe eine Hautprobe von einem Hausschwein darstellt.

6. Verfahren nach Anspruch 5, wobei die Hautprobe eine Hautprobe von einem Pietrain oder Landrace-Schwein darstellt.

7. Verfahren nach einem der Ansprüche 3 bis 6, wobei die Hautprobe eine Subkutanschicht mit einer durchschnittlichen Dicke von wenigstens 1 mm aufweist.

8. Verfahren nach einem der Ansprüche 3 bis 7, wobei die Hautprobe eine Abmessung (Länge x Breite x Dicke) von (3-6) x (3-6) x (2-5) mm aufweist.

9. Verfahren nach einem der Ansprüche 3 bis 8, wobei die zu beobachtenden Effekte die Folgenden sind:
(i) Effekte der Exposition von Substanzen auf die Hautprobe, wobei die Substanzen topisch auf die Hautprobe aufgebracht werden und/oder systemisch durch Vermischen mit einem Kulturmedium auf die Hautprobe einwirken, und/oder
(ii) Effekte der Bestrahlung der Hautprobe.

10. Verfahren nach einem der Ansprüche 3 bis 9 zur Untersuchung von:
• Modulation der Haut und/oder Haarpigmentierung;
• Modulation des Wachstums;
• Modulation der Haut und/oder Haarlebensfähigkeit und/oder Proliferation;
• Modulation des Fettmetabolismus;
• Anti-Cellulitis Eigenschaften der Substanzen;
• Gewichtsverminderung;
• Anti-Ageing Effekte, speziell durch Anregung der Fettzellen;
• Allergenes und/oder Entzündungspotential;
• UV-Schutz, speziell Vermeidung von UV Erythemen, Linderung und Heilung;
• Modulation der Bindegewebseigenschaften, speziell für die Untersuchung von Antifalteneigenschaften der Substanzen;
• Antioxidative Effekte;
• Wundheilung;
• Modulation der Hautbarrierefunktion;
• Modulation von Ionenkanälen, speziell neurofunktionalen Kanälen und insbesondere von Kanälen, die durch GABA, Glutamat, Acetylcholin, Serotonin, Adrenalin und ATP aktiviert werden sowie temperaturempfindliche Kanäle (TRPM8, TRPV3, TRPV4, TRPV1, ANKTM, TRVP2);
• Immunstimulation, Immunsuppression;
• Sebumstimulation, Sebumsuppression;
• Anti-mikrobielle Effizienz, speziell Anti-Akne Effizienz;
• Verminderung des Schweißflusses;
• Substantivität von Stoffen auf der Hautoberfläche;
• Effizienz bei der Filmbildung;
• Modulation der Haardicke;
• Feuchthaltung;
• Photoxizität;
• Hautmetabolismus von Stoffen.

11. System zur Modellierung eines ausgewählten Effektes bei der ausgewählten Behandlung von menschlicher Haut, umfassend eine ausgewählte Schweinehautprobe entnommen vom Nacken oder dem Bauch eines Schweines mit einer Haarfollikeldichte von mindestens 15 Follikeln pro cm² die mit ihrer Subkutanschicht auf einen Träger aufgebracht wird, der ein Kulturmedium und eine Probe enthält, welche eine Epidermis, eine Dermis und eine Subkutanschicht aufweist.

12. System nach Anspruch 11, wobei die Hautprobe die Hautprobe von einem Hausschwein darstellt.

## Revendications

1. Utilisation d'un échantillon de peau de porc prélevé sur le cou et/ou la partie dorsale d'un porc, présentant une densité de follicules pileux d'au moins 15 follicules au cm², permettant de simuler ou d'analyser un traitement de peau humaine ou animale, dans laquelle l'échantillon de peau de porc comprend un épiderme, un derme et une couche sous-cutanée.

2. Utilisation selon la revendication 1, dans laquelle la couche sous-cutanée comprend un follicule pileux.

3. Procédé de modélisation, de simulation ou d'analyse d'un effet sélectionné d'un traitement sélectionné de peau humaine ou animale, englobant les phases suivantes :
(a) Préparation d'un échantillon de peau prélevé sur le cou et/ou la partie dorsale d'un porc, présentant une densité de follicules pileux d'au moins 15 follicules au cm² sur une peau de porc, l'échantillon comprenant un épiderme, un derme et une couche sous-cutanée;
(b) Soumission de l'échantillon au traitement sélectionné; et
(c) Observation de l'effet du traitement de l'échantillon de peau.

4. Procédé selon la revendication 3, dans lequel l'échantillon de peau comprend au moins un follicule pileux primaire.

5. Procédé selon l'une quelconque des revendications 3 à 4, dans lequel l'échantillon de peau est un échantillon de peau de porc domestique.

6. Procédé selon la revendication 5, dans lequel l'échantillon de peau est un échantillon de peau de porc de piétrain ou de porc landrace.

7. Procédé selon l'une quelconque des revendications 3 à 6, dans lequel l'échantillon de peau comprend une couche sous-cutanée d'une épaisseur moyenne d'au moins 1 mm.

8. Procédé selon l'une quelconque des revendications 3 à 7, dans lequel l'échantillon de peau présente les dimensions suivantes (longueur x largeur x épaisseur) (3-6) x (3-6) x (2-5) mm.

9. Procédé selon l'une quelconque des revendications 3 à 8, dans lequel les effets à observer sont
(i) les effets de l'exposition de l'échantillon de peau à des substances, les substances étant appliquées localement à l'échantillon de peau et/ou par voie systémique à l'échantillon de peau par mélange avec un milieu de culture, et/ou
(ii) les effets de l'irradiation sur l'échantillon de peau.

10. Procédé selon l'une quelconque des revendications 3 à 9 pour évaluer :
• la modulation de pigmentation de la peau et/ou des cheveux
• la modulation de la pousse des cheveux;
• la modulation de la viabilité et/ou de la prolifération de la peau et/ou des cheveux;
• la modulation du métabolisme gras;
• les propriétés anti-cellulites des substances;
• l'amaigrissement;
• les effets antivieillissement, en particulier par stimulation des cellules graisseuses;
• l'irradiation et/ou le potentiel allergénique;
• la protection contre les UV, en particulier la prévention, la réduction et la cicatrisation de l'érythème solaire;
• la modulation des propriétés des tissus conjonctifs, en particulier pour évaluer les propriétés antirides de substances;
• les effets antioxydants;
• la cicatrisation des plaies;
• la modulation de la fonction barrière de la peau;
• la modulation des canaux ioniques, en particulier les canaux neurofonctionnels et de préférence les canaux activés par le GABA, le glutamate, l'acétylcholine, la sérotonine, l'adrénaline et l'ATP, et des canaux thermosensibles (TRPM8, TRPV3, TRPV4, TRPV1, ANKTM, TRVP2);
• l'immunostimulation; l'immunosuppression;
• la stimulation du sébum, la suppression du sébum;
• l'efficacité antimicrobienne, en particulier l'efficacité anti-acné;
• la diminution de la sécrétion sudorale;
• les caractéristiques de matériaux sur une surface de la peau;
• l'efficacité filmogène;
• la modulation de l'épaisseur des cheveux;
• l'hydratation;
• la phototoxicité;
• le métabolisme cutané des composés.

11. Système de modélisation d'un effet sélectionné d'un traitement sélectionné sur la peau humaine, comprenant un échantillon de peau isolé prélevé sur le cou et/ou la partie dorsale d'un porc, possédant une densité de follicules pileux d'au moins 15 follicules au cm² de peau de porc, disposé avec sa couche sous-cutanée sur un support comportant un milieu de culture, l'échantillon comprenant un épiderme, un derme et une couche sous-cutanée.

12. Système selon la revendication 11, dans lequel l'échantillon de peau est un échantillon de peau de porc domestique.
